Europäisches Patentamt

European Patent Office

Office européen des brevets

(19)

(11) Publication number: 0 222 526
A2

## EUROPEAN PATENT APPLICATION

(12)

(21) Application number: 86308117.0

(22) Date of filing: 20.10.86

(51) Int. Cl.⁴: C 12 N 15/00

(30) Priority: 29.10.85 US 792435
21.04.86 US 854139

(43) Date of publication of application:
20.05.87 Bulletin 87/21

(84) Designated Contracting States:
AT BE CH DE ES FR GB GR IT LI LU NL SE

(71) Applicant: LUBRIZOL GENETICS INC.
3375 Mitchell Lane
Boulder Colorado 80301-2244(US)

(72) Inventor: Clarke, Adrienne E.
35 Park Drive
Parkville Victoria 3052(AU)

(72) Inventor: Mau, Shaio-Lim
2 Dalkeith Close
Wheelers Hill Victoria 3150(AU)

(72) Inventor: Anderson, Marilyn
57 Higgins Avenue
Sunbury Victoria 3429(AU)

(72) Inventor: Cornish, Edwina
13/442 Cardigan Street
Carlton Victoria 3053(AU)

(72) Inventor: Tregear, Geoffrey W.
62 Hawthorn Grove
Hawthorn Victoria 3122(AU)

(72) Inventor: Crawford, Robert J.
65 Lowan Avenue
Lower Templestowe Victoria 3107(AU)

(72) Inventor: Niall, Hugh D.
552 8th Avenue
San Francisco California 94118(US)

(74) Representative: Fisher, Adrian John et al,
Carpmaels & Ransford 43 Bloomsbury Square
London WC1A 2RA(GB)

(54) Self-incompatibility gene.

(57) DNA sequences of an S-gene which encodes S-protein and controls the self-incompatibility reaction in self-incompatible plants. The DNA sequence encoding an S-protein and its attendant full-length signal sequence are provided. A method for the identification and isolation of cDNA and genomic DNA coding sequences of the S-gene are described.

# FIG. 7:

Nucleotide sequence of the near full-length cDNA coding for the 32K molecular weight $S_2$-protein of _Nicotiana alata_.

```
Pro Ala Ser Lys Pro Pro Ala Lys Leu Asp Arg Leu Gln Leu Thr Ser Val Phe Phe Ile
CCG GCA TCA AAA CCT CCC GCC AAG CTG GAC AGA CTC CAG CTA ACG TCA GTT TTC TTC ATT
-90          -80          -70          -60          -50          -40

Leu Leu Cys Ala Leu Ser Pro Ile Tyr Gly Ala Phe Glu Tyr Met Gln Leu Val Leu Thr
TTG CTT TGT GCT CTT TCA CCG ATT TAT GGG GCT TTC GAG TAT ATG CAA CTC GTG TTA ACA
-30          -20          -10          1            10           20           30

Trp Pro Ile Thr Phe Cys Arg Ile Lys His Cys Glu Arg Thr Pro Thr Asn Phe Thr Ile
TGG CCA ATC ACT TTT TGC CGC ATT AAG CAT TGC GAA AGA ACA CCA ACA AAC TTT ACG ATC
             40           50           60           70           80           90

His Gly Leu Trp Pro Asp Asn His Thr Thr Met Leu Asn Tyr Cys Asp Arg Ser Lys Pro
CAT GGG CTT TGG CCG GAT AAC CAC ACC ACA ATG CTA AAT TAC TGC GAT CGC TCC AAA CCC
             100          110          120          130          140          150

Tyr Asn Met Phe Thr Asp Gly Lys Lys Lys Asn Asp Leu Asp Glu Arg Trp Pro Asp Leu
TAT AAT ATG TTC ACG GAT GGA AAA AAA AAA AAT GAT CTG GAT GAA CGC TGG CCT GAC TTG
             160          170          180          190          200          210

Thr Lys Thr Lys Phe Asp Ser Leu Asp Lys Gln Ala Phe Trp Lys Asp Glu Tyr Val Lys
ACC AAA ACC AAA TTT GAT AGT TTG GAC AAG CAA GCT TTC TGG AAA GAC GAA TAC GTA AAG
             220          230          240          250          260          270

His Gly Thr Cys Cys Ser Asp Lys Phe Asp Arg Glu Gln Tyr Phe Asp Leu Ala Met Thr
CAT GGC ACG TGT TGT TCA GAC AAG TTT GAT CGA GAG CAA TAT TTT GAT TTA GCC ATG ACA
             280          290          300          310          320          330

Leu Arg Asp Lys Phe Asp Leu Leu Ser Ser Leu Arg Asn His Gly Ile Ser Arg Gly Phe
TTA AGA GAC AAG TTT GAT CTT TTG AGC TCT CTA AGA AAT CAC GGA ATT TCT CGT GGA TTT
             340          350          360          370          380          390

Ser Tyr Thr Val Gln Asn Leu Asn Asn Thr Ile Lys Ala Ile Thr Gly Gly Phe Pro Asn
TCT TAT ACC GTT CAA AAT CTC AAT AAC ACG ATC AAG GCC ATT ACT GGA GGG TTT CCT AAT
             400          410          420          430          440          450

Leu Thr Cys Ser Arg Leu Arg Glu Leu Lys Glu Ile Gly Ile Cys Phe Asp Glu Thr Val
CTC ACG TGC TCT AGA CTA AGG GAG CTA AAG GAG ATA GGT ATA TGT TTC GAC GAG ACG GTG
             460          470          480          490          500          510

Lys Asn Val Ile Asp Cys Pro Asn Pro Lys Thr Cys Lys Pro Thr Asn Lys Gly Val Met
AAA AAT GTG ATC GAT TGT CCT AAT CCT AAA ACG TGC AAA CCA ACA AAT AAG GGG GTT ATG
             520          530          540          550          560          570

Phe Pro ***
TTT CCA TGA TTAATAATATTTGTTTTATTGCATTATGCCATGTAAAAAAAAAATTCAAAACCTCAAGTATAAACGTG
             580          590          600          610          620          630          640

TAATCAAGACTATTAAGCACGCACTTATTGAAGACTGCACTCGGAAGAATAAGCAAAATTCTTATCAATTTATGGAAATC
             656          666          676          686          696          706          716          726

GTTATTTAAAAAAAAAAAAAAAAAAAAAGGGGGACGGACTGGGAACGGTTCTTCGGGGTCCCGG
             736          746          756          766          776          786
```

SELF-INCOMPATIBILITY GENE

Field of the Invention

This invention relates to the identification and isolation of cDNA and genomic DNA coding sequences of an S-gene which controls self-incompatibility in a wide variety of self-incompatible plants, particularly exemplified by members of the Solonaceae and Cruciferae. Studies of S-gene products, S-proteins, indicate that they are associated with the expression of the self-incompatibility genotype of such self-incompatible plants.

S-proteins are useful in control of pollen tube growth, for example as natural gametocides to control, induce or promote self-incompatibility and interspecific incompatibility. S-genes and their products can also be used in genetic manipulation of plants to create self-incompatible cultivars. Plants engineered in this way will be valuable for the economic production of hybrid seed.

Background of the Invention

Many plant species, including Nicotiana alata and Lycopersicon peruvianum, are self-incompatible, that is they cannot be fertilized by pollen from themselves or by that of a plant of the same S- (or self-incompatibility) genotype. The molecular basis of self-incompatibility is believed to arise from the presence of S-protein in the mature styles of plants; in particular, as exemplified by N. alata and L. peruvianum, S-protein has now been shown to be present in extracts of plant styles at the developmental stages of buds at first show of petal color, and at the subsequent stages of maturation of open but immature flowers, and flowers having mature glistening styles. On the other hand, S-protein is not present in the earlier developmental stages of green bud and elongated bud.

For general reviews of self-incompatibility, see de Nettancourt (1977) Incompatibility in Angiosperms, Springer-Verlag, Berlin; Heslop-Harrison (1978) Proc. Roy. Soc. London B, 202:73; Lewis (1979) N.Z. J. Bot. 17:637; Pandey (1979) N.Z. J. Bot. 17:645 and Mulcahy (1983) Science 220:1247. Self-incompatibility is defined as the inability of female hermaphrodite seed plants to produce zygotes after self-pollination. Two types of self-incompatibility, gametophytic and sporophytic, are recognized. Gametophytic incompatibility is most common and in many cases is controlled by a single nuclear gene locus (S-locus) with multiple alleles. Pollen expresses its haploid S-genotype and matings are incompatible if the S-allele expressed is the same as either of the S-alleles expressed in the diploid tissue of the pistil. During both incompatible and compatible matings, pollen tubes germinate and grow through the stigma into the transmitting tissue of the style. Tube growth from incompatible pollen grains is arrested in the upper third of the style.

In sporophytic incompatibility, pollen behavior is determined by the genotype of the pollen-producing plant. If either of the two S-alleles in the pollen parent is also present in the style, pollen tube growth is inhibited. Unlike the gametophytic systems, inhibition usually occurs at the stigma surface and not in the style. In sporophytic incompatibility, S-protein may be concentrated at or near the stigma surface. The gametophytic polyallelic system is considered to be the ancestral form of self incompatibility in flowering plants with the sporophytic system being derived from it (de Nettancourt 1977). The products of the S-gene in the two systems are considered to be structurally related.

There are five species of gametophytically self-incompatible plants and two species of sporophytically incompatible plants in which style or stigma proteins apparently related to S-genotype have been detected by either electrophoretic or immunological methods. In N. alata, an association between specific protein bands and three S-allele groups was demonstrated by isoelectric focussing of stylar extracts (Bredemeijer and Blaas (1981) Theor. Appl. Genet. 59:185). Two major antigenic components have been identified in mature styles of a Prunus avium cultivar of $S_3S_4$ genotype, one of which (S-antigen) was specific to the particular S-allele group (Raff, et al. (1981) Planta 153:125; and Mau, et al. (1982) Planta 156:505). The S-antigen, a glycoprotein, was a potent inhibitor of the _in vitro_ growth of pollen tubes from a $S_3S_4$ cultivar (Williams, _et al_. (1982) Planta 156:577). The glycoprotein was resolved into two components, purportedly representing the $S_3$ and $S_4$ products of the $S_3S_4$ genotype. Stylar protein components which have been associated with the _S_-allele group or the self-incompatibility genotype are reported in _Petunia hybrida_ (Linskens (1960) Z. Bot. 48:126), _Lilium longiflorum_ and _Trifolium pratens_ (Heslop-Harrison (1982) Ann. Bot. 49:729).

A glycoprotein corresponding to genotype $S_7$ of _Brassica campestris_ has been isolated from extracts of stigmas by gel-filtration followed by affinity chromatography and isoelectric focussing (Nishio and Hinata (1979) Jap. J. Genet. 54:307). Similar techniques were used to isolate _S_-specific glycoproteins from stigma extracts of _Brassica oleracea_ plants homozygous for _S_-alleles $S_{39}$, $S_{22}$ and $S_7$ (Nishio and Hinata (1982) Genetics 100:641). Antisera raised to each isolated _S_-specific _Brassica oleracea_ glycoprotein not only precipitated its homologous glycoprotein but also reacted with the other two _S_-specific glycoproteins of _B. oleracea_ and the $S_7$-specific glycoprotein of _B. campestris_ (Hinata _et al_. (1982) Genetics 100:649). An _S_-specific glycoprotein was isolated by Ferrari _et al_. (1981) Plant Physiol. 67:270 from a stigma extract of _B. oleracea_ using sucrose gradient sedimentation and double diffusion tests in gels in which the proteins were identified by Coomassie Blue staining. This preparation was shown to be biologically active since pretreatment of $S_2S_2$ pollen with the glycoprotein prevented the pollen from germinating on normally compatible stigmas. Recently a cDNA clone encoding part of an _S_-locus specific glycoprotein from _B. oleracea_ stigmas has been described (Nashrallah _et al_. (1985) Nature 318:263-267.

In recent work that is detailed in U.S. Patent Application Serial No. 615,079,* filed May 24, 1984, which is expressly incorporated by reference herein, stylar extracts of several self-incompatibility genotypes from both _Nicotiana alata_ and _Lycopersicon peruvianum_ were examined for the presence of _S_-gene associated protein. Glycoprotein materials were identified in the 30,000 MW region of stylar extracts of genotypes $S_1S_3$, $S_2S_3$, $S_2S_2$ and $S_3S_3$ of _N. alata_ and of genotypes $S_1S_2$, $S_2S_3$, $S_1S_3$, $S_2S_2$, $S_3S_3$ and $S_3S_4$ of _L. peruvianum_. By comparing two-dimensional gel electrophoresis of stylar extracts of the different genotypes, closely related, but distinct glycoproteins were found to segregate with the individual _S_-alleles. For

*SEE EP-A-0127467

example, the N. alata S$_2$-protein was found only in stylar extracts of the genotypes containing the S$_2$-alleles (S$_2$S$_3$ and S$_2$S$_2$). For each genotype, the genotype specific glycoprotein only appeared as the flower matured, and was detected only in stylar extracts of buds at first show of petal color and in later stages of maturation, but not in earlier bud stages. Therefore, the appearance of these glycoproteins is temporally coincident with the appearance of the self-incompatibility phenotype. The S$_2$-glycoprotein of N. alata and the S$_2$ and S$_3$-proteins of L. peruvianum were shown to be more highly concentrated in the upper style sections, which is the zone in which pollen tube inhibition occurs. Therefore, the appearance of these glycoproteins is spacially coincident with the self-incompatibility reaction. Further, corroboration of the biological activity of S$_2$-protein of N. alata was demonstrated by its inhibition of pollen tube growth in an in vitro assay (Williams, et al. (1982)).

A significant aspect of the work disclosed in U.S. Application Serial No. 615,079 was the discovery that rabbit antisera and monoclonal antibodies raised to individual S-proteins or stylar extracts showed immunological cross-reaction between S-proteins of different genotype within the same species, between S-proteins of different species and also between species having gametophytic incompatibility and sporophytic incompatibility. It was concluded therein that there is structural homology among S-proteins, and that despite apparent differences in molecular weight and pI, these proteins are a recognizable structural class in addition to their functional similarities.

U.S. Application Serial No. 615,079 also disclosed a method of purification for S-proteins which included fractionation of stylar extracts by ion exchange chromatography followed by a second fractionation by affinity chromatography. The method of purification was exemplified with the isolation of the 32K S$_2$-glycoprotein from Nicotiana alata styles.

The S-proteins that have been identified are glycoproteins, which are proteins that have been modified by covalent bonding of one or more carbohydrate groups. Little is known of the composition and structure of the carbohydrate portion of S-proteins. It is, as yet, unclear what contribution, if any, the carbohydrate portion of the S-protein makes to biological activity in the incompatibility reaction. Petunia hybrida stylar mRNA is translated in

-4-

Xenopus laevis (frog) egg cells to produce active proteins which induce the incompatibility reaction. The relative glycosylation of S-proteins produced in frog egg cells to that of the S-proteins produced in the plant is unknown; however, the post-translational processing in the foreign system is adequate to produce biologically active proteins (Donk, van der J. A. W. M., (1975) Nature 256:674-675).

Most proteins, such as the S-proteins, that are excreted from or transported within cells have signal or transit sequences that function in the translocation of the protein, for example see: Perlman, D. and Halverson, H.W., (1983) J. Mol. Biol. 167:391-409; Edens, L. et al. (1984) Cell 37:629-633.; and Messing, J. et al. in Genetic Engineering of Plants, ed. Kosuge, T. et al. (1983) Plenum Press, New York, pp. 211-227. Signal or transit DNA sequences are generally adjacent to the 5' end of the DNA encoding the mature protein, are co-transcribed with the mature protein DNA sequence into mRNA and are co-translated to give immature proteins with the signal or transit peptide attached. During the translocation process the signal or transit peptide is cleaved to produce the mature protein.

The expression of S-genes in self-incompatible plants shows very complex regulation, with S-gene products appearing in only certain tissues at certain times. The mechanism of this regulation is not yet known in detail, but involves the presence of specific regulatory DNA sequences in close proximity to the genomic DNA that encodes the S-protein. Adjacent to the structural gene and signal or transit sequences, are promoter sequences that control the initiation of transcription and exert control over protein expression levels.

Summary of the Invention

The present work describes the isolation and identification of cDNA encoding an S-gene protein and is exemplified with the $S_2$-allele associated glycoprotein of Nicotiana alata. In summary, the initial isolation involved the following general steps:

1.    isolation and purification of mature S-protein from styles;

2.    N-terminal amino acid sequencing of the mature S-protein;

3.   chemical deglycosylation of mature S-protein and identification of deglycosylated S-protein translation products in plant tissue;

4.   preparation of a cDNA library from poly($A^+$) RNA of mature styles followed by differential screening of the library with radioactively labelled cDNA from ovary and green bud style to remove non-mature style specific cDNA and obtain mature style specific clones;

5.   preparation of an S-protein specific oligonucleotide probe, based on either the amino acid sequence of the S-protein or the nucleotide sequence of a cDNA fragment produced from stylar mRNA by specific priming with mixed oligonucleotide primers which are based on the S-protein amino acid sequence.  Alternatively, the specifically primed cDNA fragment can be used directly as the S-protein probe;

6.   screening of the mature style specific clones with an S-protein specific DNA probe to obtain full or nearly full length clones which encode the S-protein and its attendant signal or transit sequence.

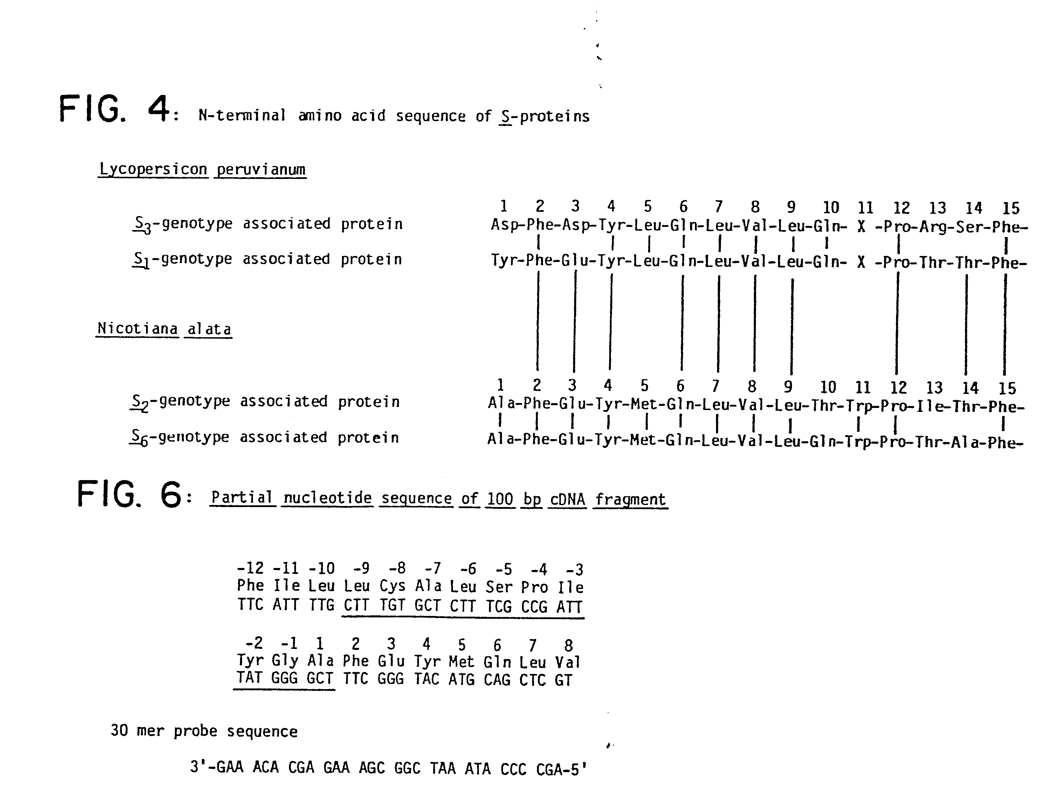

It is an important aspect of this invention that a nearly full length N. alata $S_2$-protein cDNA clone, obtained as summarized above, hybridizes in Northern blot experiments with N. alata mature style poly($A^+$) RNA not only from the $S_2S_2$ and $S_2S_3$ genotypes, but also from the $S_1S_3$ and $S_3S_3$ genotypes. Further, the N. alata $S_2$-protein specific clone also hybridizes with style mRNA from L. peruvianum (genotype $S_1S_3$) and B. oleracea (mixed genotype). These observations demonstrate that substantial DNA homology exists among S-genes in addition to the protein homology evidenced by the existence of immunological cross-reaction among S-proteins.  A further confirmation of substantial amino acid sequence homology among S-proteins is shown herein by a comparison of the N-terminal amino acid sequence of the N. alata $S_2$-protein with the N. alata $S_6$-protein and the L. peruvianum $S_1$ and $S_3$-proteins.  In the region sequenced (amino acids 1-15, Figure 4), the N. alata $S_2$-protein is 80% homologous to the N. alata $S_6$-protein, 67% homologous to the L. peruvianum $S_1$-protein, and 53% homologous to the L. peruvianum $S_3$-protein.

A further aspect of this invention is the use of the nearly full length N. alata S$_2$-protein specific cDNA clone as a hybridization probe for the isolation and identification of cDNA clones specific for other S-proteins. Although the procedure summarized above (steps 1-6) is generally applicable to any S-protein, a major improvement to the procedure is the use of the N. alata S$_2$-protein clone in step 6, as a probe in a hybridization screen to detect clones which contain S-protein specific cDNA. The availability of the S$_2$-protein specific probe obviates the need to isolate each individual S-protein, determine the N-terminal protein sequence and prepare a unique probe for each S-protein. The use of the S$_2$-specific cDNA clone will greatly facilitate the isolation of cDNA clones for other S-proteins, particularly when the limited availability of pure S-protein makes protein sequencing difficult if not impossible. There are several recent examples of the application of cDNA hybridization probes for the identification of other cDNA clones which encode homologous proteins. The basic procedures employed are described in Staswich, P. and Chrispeels, M. (1984) J. Molecular Appl. Genet. 2:525; Dennis, E. S. et al. (1984) Nucleic Acids Research 12:3983; and Dennis, E. S. et al. (1985) Nucleic Acids Research 13:727, for example.

A full-length cDNA clone of the N. alata S$_2$-gene was obtained by hybridization screening using the nearly-full length N. alata S$_2$-cDNA as a probe. This full-length clone contains a sequence that encodes the mature S$_2$-protein as well as the complete signal or transit polypeptide sequence. This signal sequence functions in the extracellular translocation of the mature S-protein from the transmitting tract cells. The transmitting tract is the tissue through which the pollen tubes grow on their way to the ovary, and it is in this tissue that the S-gene is expected to be expressed.

Signal or transit DNA sequences are useful in combination with the adjacent DNA sequences of the mature protein in affecting the excretion or translocation of the mature protein in heterologous expression systems. Further, the presence of signal or transit DNA sequences have been shown to enhance protein expression levels (Edens, L. et al., 1984). Signal or transit sequences are useful in the construction of chimaeric genes in which they are fused to a heterologous protein, for example in a recombinant vector to direct translocation of the protein. Examples of such heterologous fusions are found in prokaryotic systems, Palva, I. et al., (1982) Proc. Natl. Acad. Sci. USA 79:5582-5586 and Palva I. et al., (1983) Gene 22:229-235; and eukaryotic systems, Edens, L. et al. (1984) and Rose, M. et al. (1981) Proc. Natl. Acad. Sci USA 78:2460-2464.

Plant signal or transit sequences are particularly important for use in combination with their adjacent DNA sequences or in chimaeric gene fusions with heterologous proteins to target mature protein to specific organelles in plant cells or for excretion from the cell. (Van den Bioeck, G. et al., (1985) Nature 313:358-363). For example, such plant chimaeras can be used for the targeting of proteins toxic to plant pathogens or of gene products associated with pesticide resistance in plant tissue.

A full or nearly full-length cDNA clone coding for the N. alata $S_2$-protein, is also useful as a probe of a style genomic library in order to isolate and identify a chromosomal gene encoding an S-protein. In addition to the mature protein sequence and signal or transit sequence, a genomic clone can be obtained which includes the promoter sequences involved in regulating the timing and rate of transcription and expression of the $S_2$-protein. The screening of genomic libraries with cDNA probes is well known in the art. The basic procedures as applied to plant genes are described in Hoffman, L. M. (1984) J. Mol. Genet. 2:447-453 and Mazure, B. J. and Chui, C.-F. (1985) Nucleic Acids Research 13:2373, for example.

The S-protein DNA coding sequences that are isolated by the techniques described herein can be used in heterologous in vivo expression systems to direct synthesis of S-protein which can thereby be produced in significant amounts in biologically active form to be used, for example, as natural gametocides. The DNA sequence encoding the mature S-protein can be so employed separately or in combination with its attendant regulatory sequences.

Promoter sequences are likewise useful in combination with DNA sequences encoding proteins in effecting the transcription of DNA sequences and exerting control over protein expression levels in heterologous expression systems. Plant promoter sequences are particularly useful for the expression of heterologous proteins in plant cells, since promoters derived from prokaryotes or non-plant eukaryotes may not function efficiently in plant cells.

0222526

The present invention provides novel genetic constructs containing DNA encoding an $\underline{S}$-protein associated with the self-incompatibility phenotype. Knowledge of the amino acid sequence of the mature $\underline{S_2}$-protein and the successful development of a differential screening procedure were important factors in the initial isolation of the cDNA clone. Hybridization experiments and amino acid sequence data have confirmed the existence of amino acid and nucleotide sequence homology among $\underline{S}$-proteins and demonstrate that the $\underline{S_2}$- protein specific clone can be employed as a hybridization probe in the isolation of other $\underline{S}$-genes.

<u>Detailed Description of the Invention</u>

Details of the isolation and identification of cDNA clones specific to $\underline{S}$- proteins and the use of full or nearly full length cDNA clones specific to the $\underline{N.\ alata}$ $\underline{S_2}$-protein as hybridization probes are given in the following detailed description and in the accompanying drawings:

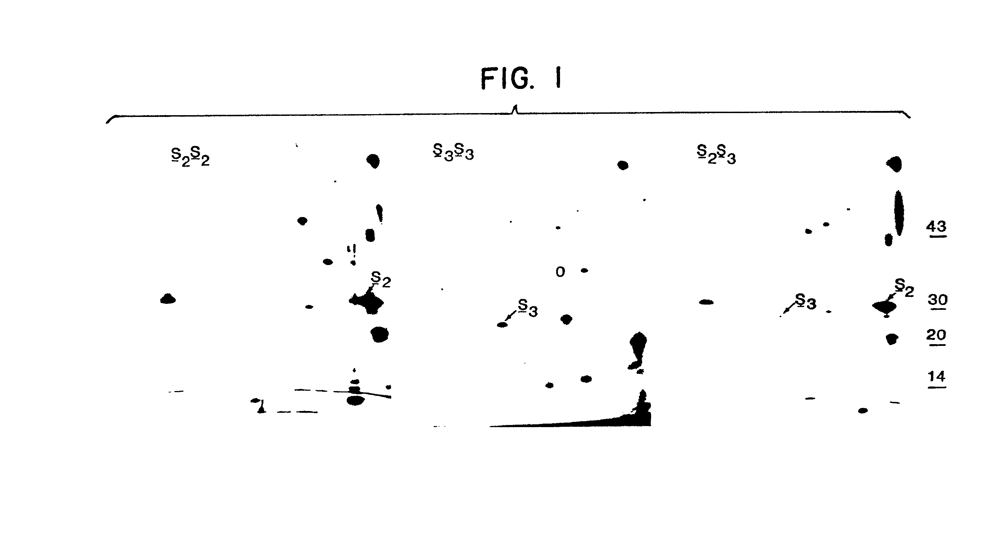

Figure 1 illustrates the separation of stylar extracts of $\underline{N.\ alata}$ genotypes $\underline{S_2S_2}$, $\underline{S_2S_3}$ and $\underline{S_3S_3}$ by selected 2-dimensional gel electrophoresis. The protein bands associated with the two alleles are identified.

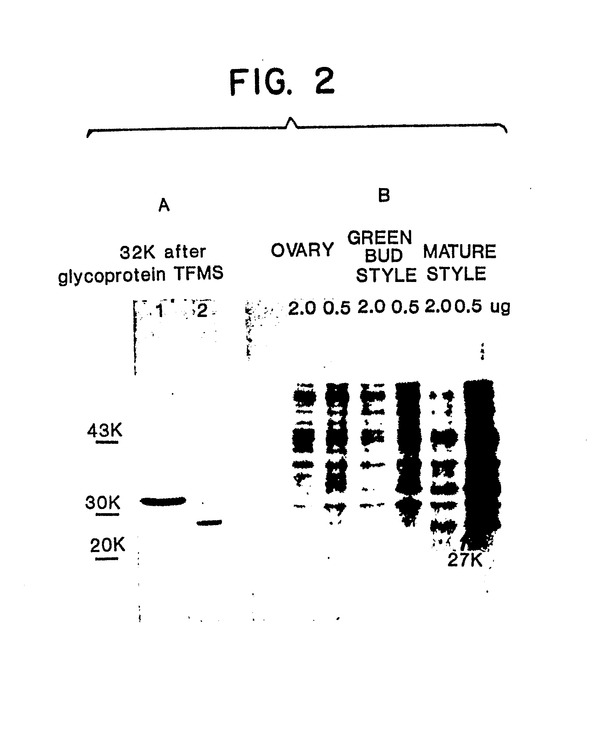

Figure 2 provides a comparison of (a) the chemically deglycosylated mature $\underline{S_2}$ glycoprotein of $\underline{N.\ alata}$ of molecular weight 26 kd, with the (b) $\underline{in\ vitro}$ translation products of style poly($A^+$) RNA, by SDS-gel electrophoresis. Note the presence of the 27K kd molecular weight protein band only in the translation products from mature style poly($A^+$) RNA. The 27 kd molecular weight translation product is slightly larger than the chemically deglycosylated mature $\underline{S_2}$ protein, consistent with the presence of a signal sequence in the 27 kd protein.

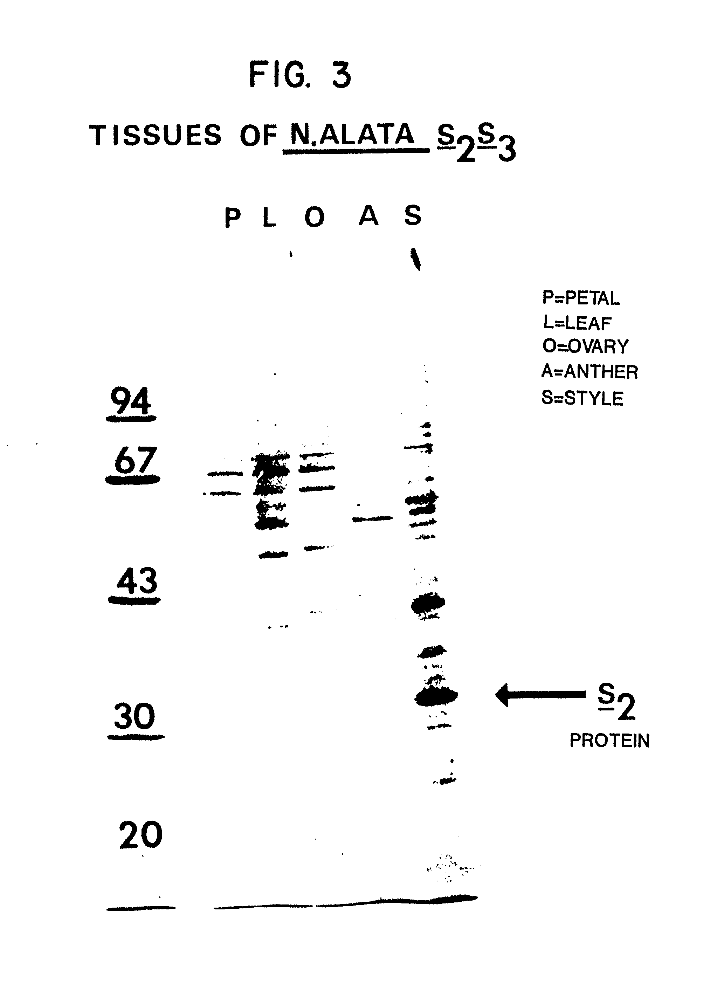

Figure 3 presents a comparison of the SDS-polyacrylamide gel electrophoresis of protein extracts from ovary, style and other $\underline{N.\ alata}$ ($\underline{S_2S_3}$) tissue. There is more similarity between the extracts of ovary and style than between extracts of other organs and style, as shown by the protein bands visualized by Coomassie Blue staining.

Figure 4 provides the N-terminal amino acid sequences of the mature $\underline{N.\ alata}$ $\underline{S_2}$- and $\underline{S_6}$-proteins and the mature $\underline{L.\ peruvianum}$ $\underline{S_1}$ and $\underline{S_3}$ proteins. Amino acid homology is indicated by solid lines.

9-

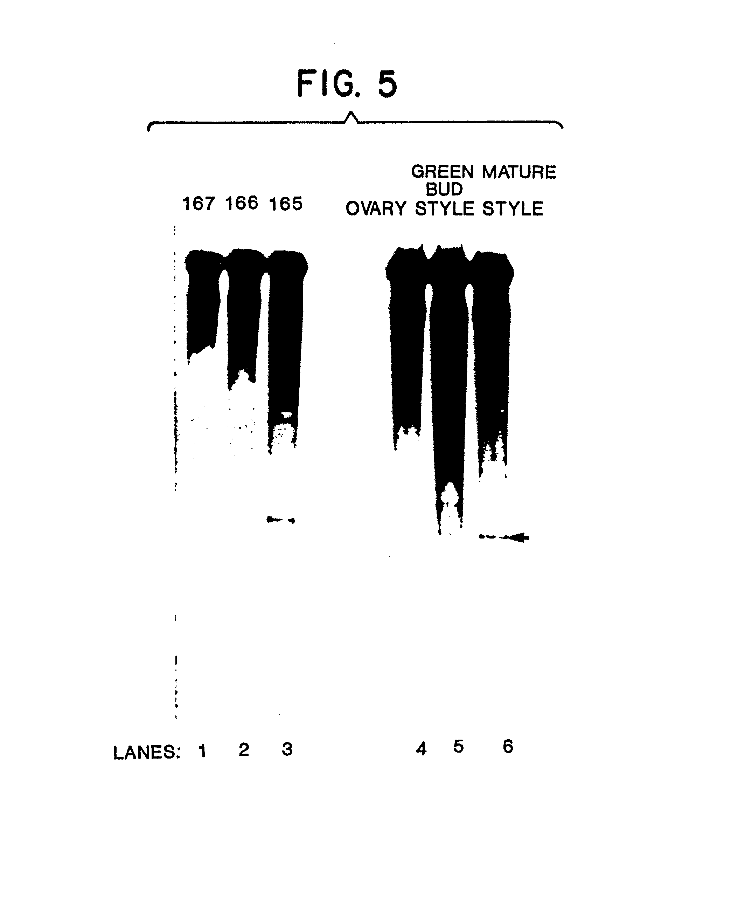

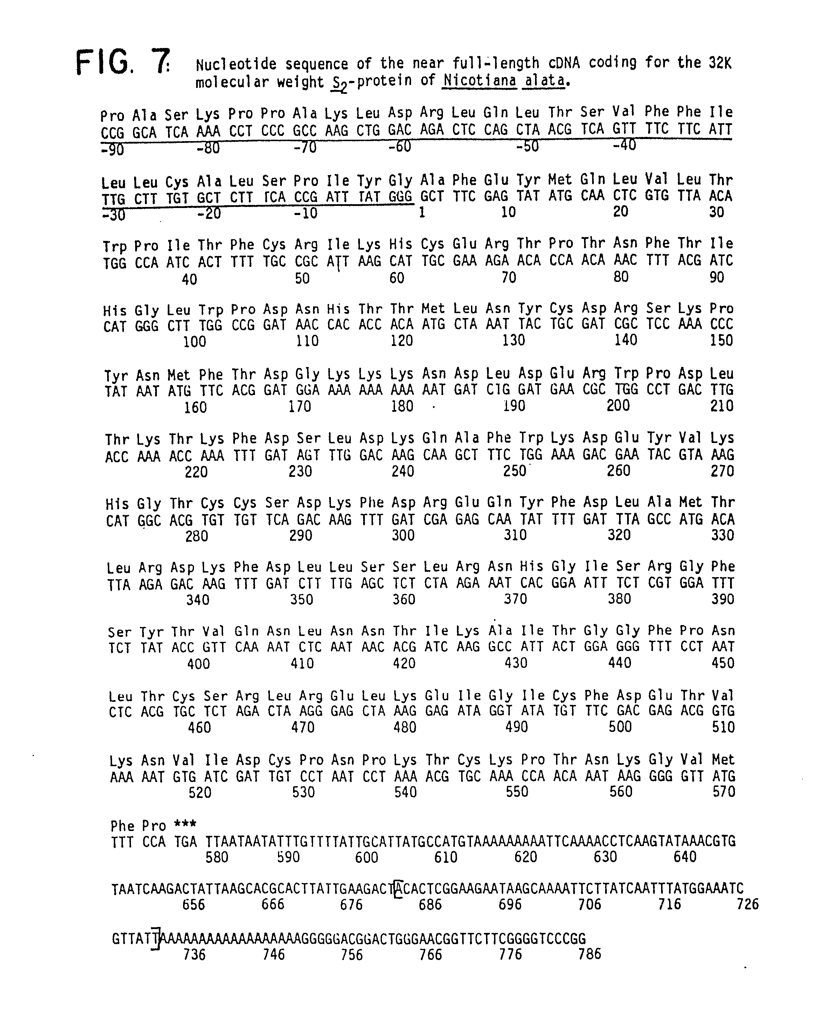

Figure 5 shows the production of a 100 bp cDNA fragment from mature style poly($A^+$) RNA using synthetic oligonucleotide 14-mers as primers. One batch primed synthesis of a single 100 bp fragment (tracks 1, 2 and 3). Tracks 4, 5, and 6 show that only the 100 bp fragment is produced with mature style poly ($A^+$) RNA when pooled synthetic primers are used. Only traces of the 100 bp fragment are detected from ovary and green bud style poly ($A^+$) RNA.

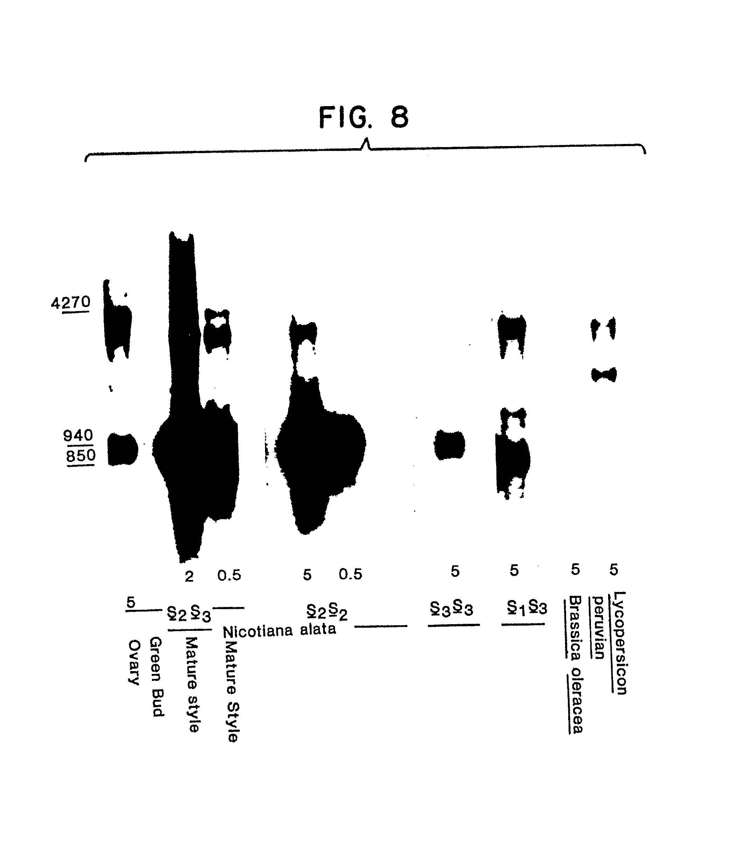

Figure 6 provides the partial nucleotide sequence of the $S_2$-protein specific 100 bp cDNA fragment on which the preparation of the 30-mer synthetic probe was based. Nucleotides on the 5' side of residue 1 of the mature $S$-protein are indicated by negative numbers. The deduced amino acid sequence is shown above the nucleotide sequence. The probe sequence is also provided, and is complementary to the underlined nucleotide sequence. A reading error at nucleotide 24 (amino acid -5) was later discovered. The correct nucleotide at position 24 is A.

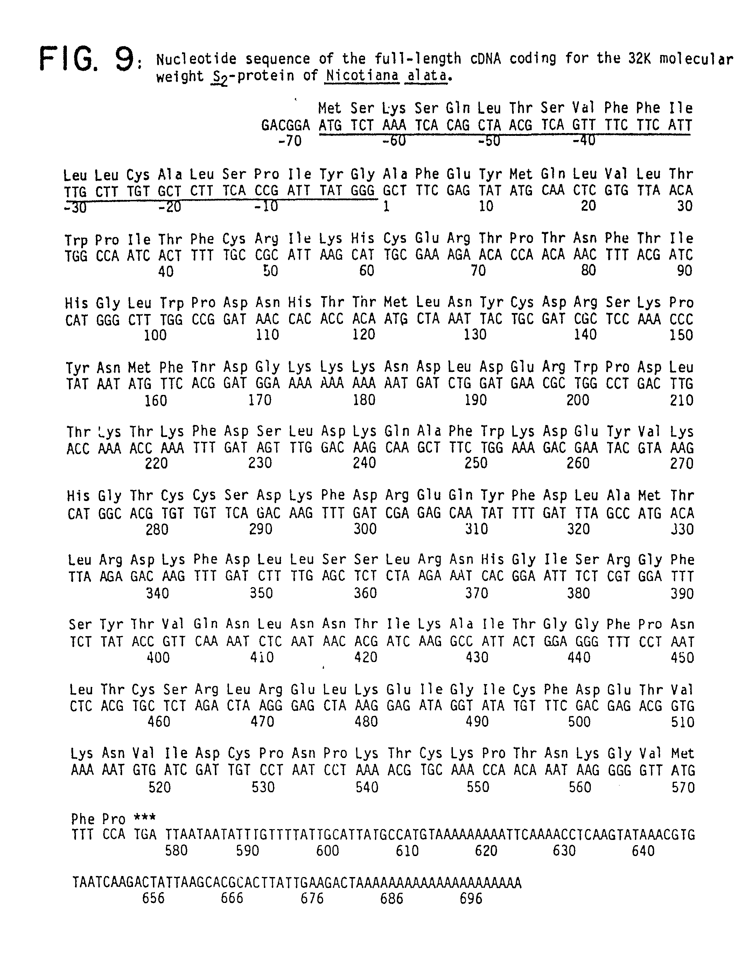

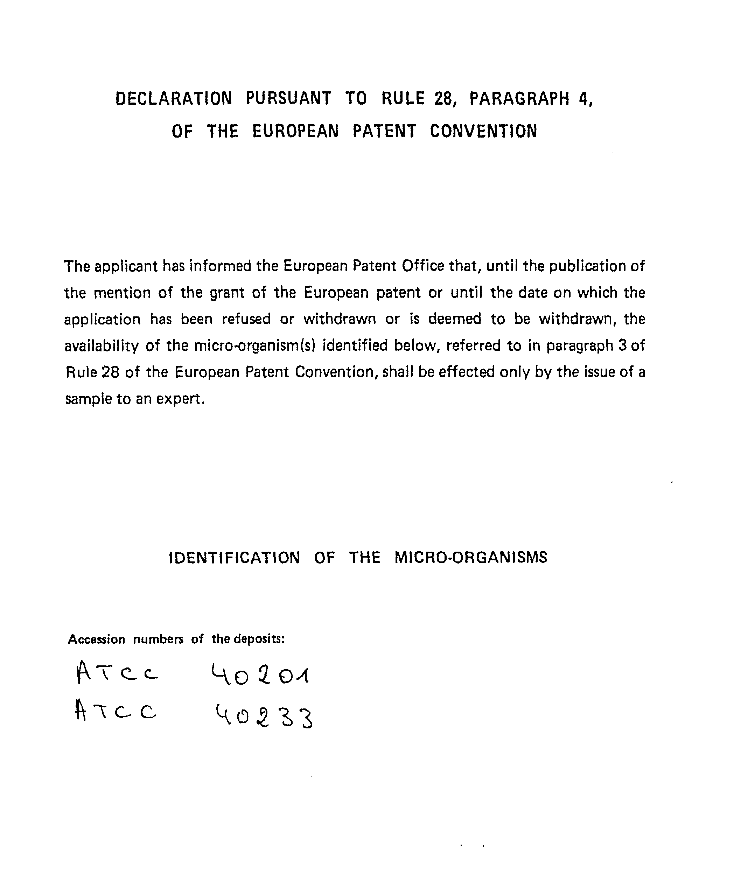

Figure 7 provides the nucleotide sequence of the near full-length cDNA coding for the 32K molecular weight-$S_2$-protein. cDNA contains the full mature protein encoding sequence and part of the signal encoding sequence. Nucleotide residues are numbered in the 5' to 3' direction. Nucleotides on the 5' side of residue 1 of the mature $S$-protein are indicated by negative numbers. The deduced amino acid sequence is shown above the nucleotide sequence. The partial signal sequence is underlined.

Figure 8 is a Northern blot analysis of mature style poly($A^+$) RNA from N. alata genotypes $S_3S_3$, $S_1S_3$, $S_2S_2$ and $S_2S_3$, L. peruvianum genotypes $S_1S_3$ and mixed genotypes from B. oleracea. Poly($A^+$) RNA from N. alata $S_2S_3$ green bud style and ovary are also included. All tracks are probed with $^{32}$P-labelled probe from the NA-2-1 clone cDNA insert encoding the N. alata $S_2$-protein described infra.

Figure 9 provides the nucleotide sequence of the full-length cDNA coding for the 32K molecular weight $S_2$-protein. This cDNA contains the full mature protein encoding sequence and the full signal encoding sequence. Nucleotide residues are numbered in the 5' to 3' direction. Nucleotides on the 5' side of residue 1 of the mature $S$-protein are indicated by negative numbers. The deduced amino acid sequence is shown above the nucleotide sequence. The signal sequence is underlined.

The following definitions apply in the specification and claims: The _S-gene protein is the product of the _S-gene or _S-allele. The term protein as used herein also includes glycoprotein. Although the biochemical mechanism of the self-incompatibility reaction is not fully understood, the _S-protein is associated with the presence of self-incompatibility. Accordingly, the _S-protein must (1) show segregation with the _S-allele; (2) be localized in the tissue where the incompatibility reaction is localized and (3) occur in the appropriate plant tissue in coincidence with the expression of self-incompatibility. In addition, it will be understood that the biological activity of the _S-protein in an _in vitro_ assay will provide corroboration that the _S-protein is itself functionally active for pollen inhibition. However, it is possible that the active component is a modified protein or a secondary product. In such cases, biological activity of the _S-protein may require the activity of other components in order to be manifested in a bio-assay system. A mature _S-protein is the processed form of the _S-protein from which the signal or transit peptide has been cleaved. This is the form of the protein isolated from stylar tissue.

The _S-gene or _S-allele contains the DNA coding sequences for the mature _S-proteins defined above. Further, the _S-gene contains the coding region for a signal or transit peptide and other information necessary to the translation and processing of the _S-protein. Further, the _S-gene is likely to contain promoter sequences involved in the transcription and expression and processing of the _S-protein. A full length cDNA clone comprises the DNA sequence encoding a mature protein and the entire regulatory signal or transit sequence.

The term recombinant vector is used herein to designate a DNA molecule capable of autonomous replication in a host eukaryotic or prokaryotic cell, into which heterologous DNA sequences can be inserted, so that the heterologous sequences are replicated in the host cell. Conventional techniques known to those of ordinary skill in the art are used to introduce the vector into its host cell (Maniatis et al., 1982). Recombinant vectors often contain a marker displaying a selectable phenotype such as antibiotic resistance to allow selection of transformed cells.

-11-

A DNA molecule that is substantially pure will migrate as a single band in agarose or polyacrylamide gel electrophoresis, using conventional procedures described in Maniatis et al. (1982) and exemplified in Figure 5.

The term homology is used in the art to describe a degree of amino acid or nucleotide sequence identity between polypeptides or polynucleotides. The presence of sequence homology is often used to support a genetic or functional relationship between polypeptides or nucleotide sequences. The presence of amino acid sequence homology between polypeptides implies homology between the DNA sequences that encode the individual polypeptides. Since the genetic code is degenerate the degree of homology between polypeptides or proteins is not necessarily the same as that between the DNA sequences that encode them. The degree of homology between polypeptides or polynucleotides can be quantitatively determined as a percent homology if the sequences are known. In the absence of sequence information for comparison, the presence of homology is usually determined operationally by experiment. In the case of DNA or RNA sequences, hybridization experiments are used to determine the presence or absence of homology. Since the strength of a particular hybridization signal depends on the experimental conditions used as well as the degree of homology, it is convenient to define homology in relation to the experimental conditions used. We use the term substantially homologous as the degree of homology that must exist between the hybridization probe and a target RNA or DNA sequence in order to select the target sequence from a background of undesired sequences using hybridization experiments as described herein. Based on our knowledge of the degree of amino acid homology between the $S_2$ protein of N. alata and other S-proteins, substantial homology is herein quantitatively defined as equal to or greater than about 53% homology.

The term cDNA is understood in the art to denote the single stranded complementary DNA copy made by action of reverse transcriptase on an mRNA template. Herein, the term cDNA is also used to denote any single or double stranded DNA that is replicated from this first complementary copy. cDNA coding sequences are distinguished from genomic DNA sequences by the potential presence of intron non-coding sequences in the genomic DNA. In vivo, introns are removed from messenger RNA by splicing events that produce mature mRNA. It is mature mRNA that is used in the initial preparation of cDNA by reverse transcription.

-12-

The term recombinant DNA molecule is used herein to distinguish DNA molecules in which heterologous DNA sequences have been artificially ligated together by the techniques of genetic engineering, for example by _in vitro_ ligation using DNA ligase (Maniatis, T. _et al._ (1982) _Molecular Cloning_, Cold Spring Harbor Laboratory, Cold Spring Harbor, New York). Heterologous DNA sequences are derived from different genetic entities.

The process of cloning a DNA fragment involves excision and isolation of the DNA fragment from its natural source, insertion of the DNA fragment into a recombinant vector and incorporation of the vector into a microorganism or cell where the vector and inserted DNA fragment are replicated during proliferation of the microorganism or cell. The term clone is used to designate an exact copy of a particular DNA fragment. The term is also used to designate both the microorganism or cell into which heterologous DNA fragments are initially inserted and the line of genetically identical organism or cells that are derived therefrom.

The plant materials used in the present work were based on _Nicotiana alata_ self incompatible heterozygous genotypes $S_1S_3$ and $S_2S_3$ (Dr. K. R. T. Pandey, Genetics Unit, Grasslands Division, DSIR Palmerston North, New Zealand) and on _L. peruvianum_ heterozygous genotypes $S_1S_2$ and $S_1S_3$ (Victorian State Department of Agriculture, Burnley, Victoria, Australia) and _Brassica oleracea_ mixed genotype. Self-incompatibility genotype was confirmed by hand pollination. Homozygous self-incompatibility genotypes were generated, as described in U.S. Patent Application Serial No. 615,079, by self-pollination of flower buds at the early bud stage before expression of self-incompatibility.

Mature non-pollinated styles were obtained from flowers that had been emasculated at the onset of petal coloration or from yellow buds. These mature styles were removed and used immediately or stored at -70°C. Styles refer to stigmas and style which were excised together. Ovary was separated from styles. Green bud styles refer to immature styles before the onset of self-incompatibility.

Except as noted hereafter, standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in: Maniatis et al. (1982) Molecular Cloning, Cold Spring Harbor Laboratory, Cold Spring Harbor, New York; Wu (ed.) (1979) Meth. Enzymol. 68; Wu et al. (eds.) (1983) Meth. Enzymol. 100 and 101; Grossman and Moldave (eds.) Meth. Enzymol. 65; Miller (ed.) (1972) Experiments in Molecular Genetics, Cold Spring Harbor Laboratory, Cold Spring Harbor, New York; Old and Primrose (1981) Principles of Gene Manipulation, University of California Press, Berkeley; Schleif and Wensink (1982) Practical Methods in Molecular Biology; Glover (ed.) (1985) DNA Cloning Vol. I and II, IRL Press, Oxford, UK; Hames and Higgins (eds.) (1985) Nucleic Acid Hybridization, IRL Press, Oxford, UK; Sellow and Hollaender (1979) Genetic Engineering: Principles and Methods, Vols. 1-4, Plenum Press, New York, which are expressly incorporated by reference herein. Abbreviations and nomenclature, where employed, are deemed standard in the field and commonly used in professional journals such as those cited herein.

Isolation of cDNA encoding the 32K $S_2$-gene protein of N. alata

A method for isolating and purifying the S-gene associated glycoproteins from mature styles had been established using a combination of ion exchange and affinity chromatography (U.S. Patent Application Serial No. 615,079). This method had been applied to the isolation and purification of N. alata $S_2$-protein. More recently, purified protein yield improvements have been obtained by using a less basic buffer (pH 7 rather than pH 7.8) in affinity chromatography. The S-protein appears to be more stable at lower pH. As illustrated in Fig. 1, it was possible to isolate a single component of MW 32K associated with the $S_2$-allele of Nicotiana alata. Chemical deglycosylation of this component yielded a single product of approximately 26 kd in molecular weight, shown in Figure 2a. The results of in vitro translation of mRNA from mature styles, green bud style and ovary are shown in Fig. 2b. Total RNA was isolated by conventional methods. Since most mRNA is polyadenylated, poly(dT) cellulose chromatography was used to isolate mRNA, as poly($A^+$) RNA. The various poly($A^+$) RNA fractions were translated using an amino acid depleted rabbit reticulocyte lysate kit (Amersham No. N.150, Arlington Heights, Ill.) in the presence of tritiated amino acids. An in vitro translation product of approximately 27 kd molecular weight is detected only from mature style mRNA.  This product is slightly larger than the chemically deglycosylated protein.  It is therefore identified as the full length immature $S_2$-protein, which is composed of mature $S_2$-protein and its signal peptide.

Based on this finding, a protocol of differential screening was adopted as the initial part of the strategy to isolate cDNA coding for $S_2$-protein.  A cDNA library was prepared in λgt10 phage using mature style poly($A^+$) RNA of N. alata genotype $S_2S_3$.  Mature style poly($A^+$) RNA was transcribed into double stranded cDNA by conventional methods (Maniatis et al., 1982).  End-repair, EcoRI methylation and EcoRI linker ligation reactions were carried out and the cDNA was cloned into the EcoRI site of the λgt10 vector (Huynh, T. et al. (1985) in Practical Approaches in Biochemistry, DNA Cloning Vol. 1 ed. Glover, D. IRL Oxford, pp. 49-78).  This library was subjected to differential screening using $^{32}$P-labelled cDNA from mature and green bud styles.  The lambda-phage was used to infect Escherichia coli C600 cells.  Plaques that hybridized strongly only to the mature style cDNA were selected and differentially screened a second time using $^{32}$P-labelled cDNA prepared from either mature style or ovary mRNA.  Again plaques that hybridized strongly only to the mature style cDNA were selected.  Ovary cDNA was used in this second screen because SDS-gel electrophoresis indicated that extracts of mature style and ovary had some common proteins which were not expressed in green bud styles (Figure 3).  Surprisingly, tissues other than ovary and green bud were found to be unsuitable sources of cDNA for differential screening since the protein profiles of other organs were found to be too diverse from that of mature style to be useful.  Therefore, differential screening with ovary and green bud cDNA, although considerably less convenient, was necessary to discriminate mature style-specific cDNA.  The resultant cDNA clones were specific for mature style.

Once the cDNA mature style library had been differentially screened, a $S_2$-protein specific DNA probe was required for final screening of the clone library.  The first step in the preparation of the probe was the determination of the N-terminal amino acid sequence of the N. alata $S_2$-protein (Figure 4).  Conventional microsequencing techniques were used (Hewick, R.M. et al. (1981) J. Biol. Chem 256:7990-7997).  The several alternative methods of screening the mature style specific library (cited supra) rely on a knowledge of the amino acid sequence.  S-protein is made in minuscule amounts at limited times and in limited tissue. Several hundred styles must be dissected from the flowers in order to obtain sufficient pure S-protein for micro-amino acid sequencing. Consequently only short segments of N-terminal sequence could be determined with the amounts of material available for analysis, using conventional microsequencing techniques. Unfortunately, the N-terminal amino acid sequence proved to have highly redundant coding oligonucleotide possibilities. Nevertheless, a partial-length cDNA was isolated by the following procedure. A set of synthetic mixed oligonucleotide primers were prepared based on the partial amino acid sequence. A set of 24 14-mers, covering all the codon ambiguities at amino acids 4-8, was synthesized. These synthetic mixed oligonucleotides were then used in three batches of eight 14-mers each, to prime synthesis of cDNA from N. alata ($S_2S_3$) mature style poly($A^+$) RNA.

As shown in Figure 5, only one batch (No. 165) was found to be specific for the priming reaction. Surprisingly, a single cDNA band 100 nucleotides in length was identified in this reaction. A 100 bp-nucleotide band was also observed when the pooled 14-mers were used to prime poly ($A^+$) RNA from mature styles; only traces of this fragment were detected in priming from ovary or green bud style mRNA.

The 100 nucleotide long band was eluted from an acrylamide gel and sequenced yielding the $S_2$-protein coding sequence from amino acid -12 in the signal sequence, up to amino acid 2 of the mature protein, as shown in Figure 6. From this sequence a single 30-mer was synthesized which covered the part of the signal sequence to -9 and included the first amino acid codon of the coding sequence (Figure 6). This amino acid region was chosen in order to insure that the synthetic probe would identify cDNA clones that extended into the signal sequence codons. This strategy was adopted for convenience, since adequately large amounts of the synthetic probe could be prepared in a single synthesis. Alternatively, the 100 bp fragment could have been cloned, amplified, purified and radioactively labelled for use as a probe.

The 30-mer was used as an $S_2$-protein specific probe to screen the mature style-specific clones previously identified by differential screening. One of the clones obtained was chosen for further study. The clone, designated NA-2-1, contained a cDNA insert of 877 bp which could be excised as a single

-16-

fragment from the lambda vector by EcoRI digestion. The 877bp insert has been cloned into M13 phage (M13mp8) and has been deposited with the American Type Culture Collection, 12301 Parklawn Drive, Rockville, Maryland 20852 (Accession No. 40201).

The nucleotide sequence of the 877bp cDNA insert from NA-2-1 for the 32K style glycoprotein associated with the $S_2$-allele of N. alata is given in Figure 7. Nucleotide residues are numbered in the 5' to 3' direction. The deduced amino acid sequence of the protein is shown above the nucleotide sequence and the partial signal sequence is underlined.

In sequencing of the NA-2-1 insert, a stop codon was identified in the middle of what was believed to be the protein coding sequence. Protein sequencing of the polypeptide fragment corresponding to the coding region in question revealed that an extra adenine nucleotide had been inserted in the region 171-182 (Fig. 7) of the clone, most likely the result of a cloning artifact. The extra adenine nucleotide has not been included in the sequence of Figure 7. The amino acid sequence of nucleotides 1-30 of the NA-2-1 insert sequence is identical to the N-terminal sequence of the first 10 amino acids of the mature $S_2$ protein. With exclusion of the extra adenine nucleotide, the mature protein coding sequence extends to a TGA stop codon (577-579) and encodes a 192 amino acid mature protein. The cDNA insert from NA-2-1 extends 171 nucleotides from this TGA stop codon and includes a poly(A$^+$) tail 18 residues long. The sequence following the poly(A$^+$) tail is also believed to be the result of a cloning artifact. The partial signal sequence that is included by the NA-2-1 insert has the typical features described for eukaryotic signal sequences (von Heijne, G. (1983) Eur. J. Biochem. 133:17-21; von Heijne, G. (1985) J. Mol. Biol. 184:99-105) with a relatively hydrophylic sequence of 5 amino acids at the C-terminal end, an extremely hydrophobic sequence (-6 to -14 amino acids) and a more hydrophylic sequence an the N-terminal end.

The particular clone insert chosen for sequencing (NA-2-1) does not extend in the 5' direction to an ATG initiation codon, so it is not believed to contain the full signal sequence. It would be a matter of ordinary skill in the art, however, to use the 30-mer probe or the NA-2-1 insert as a probe to isolate the full length clone with the complete signal sequence.

A full-length clone was obtained from a second cDNA library which had been prepared using a method (Okayama et al. (1982) Mol. Cell Biol. 2:161-170) which optimizes the recovery of full length clones. This library was screened with the 30-mer probe as well as with the cDNA insert from clone NA-2-1 (described above). A clone designated NA-2-2 was obtained which hybridized to both probes. Figure 9 shows the nucleotide sequence of the cDNA insert from NA-2-2. The NA-2-2 clone insert has been subcloned into M13 phage (M13mp8), designated pAEC9, and has been deposited with the American Type Culture Collection, 12301 Parklawn Drive, Rockville, Maryland 20852 on April 16, 1986, and has been given Accession No. 40233.

The sequence of the cDNA insert of clone NA-2-2 (Figure 9) includes an ATG at its 5' end that is a potential initiation codon and exactly matches the sequence of NA-2-1 (Figure 7) from nucleotide -54 to 682 of the NA-2-2 cDNA. The sequence contains an open reading frame of 642 bp which encodes a protein with a predicted molecular weight of 24,847 that includes a putative signal sequence of 22 amino acids. The sequence of Figure 9 encodes the mature $S_2$ protein with a signal sequence that would direct the extracellular transfer of the $S_2$ glycoprotein from the transmitting tract cells. The full-length signal sequence has the typical features described for eukaryotic signal sequences (von Heijne, 1983, and von Heijne, 1985).

Apart from the differences at the 5' end, clones NA-2-1 and NA-2-2 also differ in the length of their 3' untranslated sequence. They are identical to nucleotide 662, which is the polyadenylation site in clone NA-2-2. The clone insert from NA-2-1 has an additional 50 nucleotides of untranslated mRNA and a polyA tail of 18 residues. This difference at the 3' end suggests that there are alternative polyadenylation sites in $S_2$ RNA transcripts.

Recently, a cDNA clone encoding part (about 30%) of an S-locus specific glycoprotein from B. oleracea has been described (Nashrallah et al., 1985). This clone is reported to represent the 3' end of the coding sequence of the B. oleracea S-protein. There is no apparent homology between this partial sequence and that of the N. alata $S_2$-cDNA (Figure 7 and 9). The sequence homology between N. alata and Brassica S-genes indicated by Southern blots may then reside in the as yet undescribed 70% of the Brassica oleracea S-protein clone.

It will be obvious to one of ordinary skill in the art that the DNA sequence information provided herein can be used for the chemical synthesis of oligonucleotide probes that can be used in the hybridization screens described herein. See, for example, Caruthers, M.H. (1984) Contemp. Top. Polym. Sci. 5:55-71; Eisenbeis, S.J. et al. (1985) Proc. Natl. Acad. Sci. USA 82:1084-1088.

Hybridization of the N. alata $S_2$-protein cDNA clone to poly($A^+$) RNA from mature styles of N. alata, L. peruvianum and Brassica oleracea

A $^{32}$P-labelled copy of the cDNA insert from the NA-2-1 clone, which contains the $S_2$-protein coding region, was used in Northern blot hybridization experiments with poly($A^+$) RNA prepared from mature styles of N. alata genotypes $S_1S_3$, $S_2S_3$, $S_2S_2$ and $S_3S_3$, as well as mature styles of L. peruvianum genotype $S_1S_3$ and green bud styles and ovaries of N. alata genotype $S_2S_3$, Figure 8. The size of the major transcript in mature styles bearing the $S_2$-allele was 940 bases, based on comparison to 5' end labelled-HindIII-EcoRI markers, with two minor transcripts at 1500 and 3500 bp. The 940 base transcript was also present in RNA from $S_3S_3$ and $S_1S_3$ styles but at a much reduced frequency, that is 1% or less than the level in $S_2S_2$ or $S_2S_3$ styles. The major transcript was not present in green bud RNA but was detected in RNA from ovaries of mature flowers, again at a much lower concentration than that of mature styles (less than 1%).

Lycopersicon peruvianum genotype $S_1S_3$ contains readily detectable levels of a 2.5 kb mRNA that hybridizes with the NA-2-1 cDNA insert. The $S_1$ and $S_3$ proteins from L. peruvianum both have estimated molecular weights of 28 kd; the RNA blot analysis indicates that the mRNA transcripts encoding these proteins are identical in size. Hybridization with Brassica oleracea mature style mRNA was faint under the conditions used. However, Southern blots of restricted genomic DNA from leaves of Brassica campestris show bands which strongly hybridize to the NA-2-1 clone insert.

These results indicate homology between the DNA coding sequences of the N. alata $S_1$ and $S_3$ proteins and the $S_2$ protein of N. alata. Further, they indicate that there is homology between the coding sequences of the N. alata $S_2$ protein and those of Lycopersicon peruvianum $S_1$ and $S_3$ protein. The weak

-19-

hybridization of the $S_2$-protein cDNA probe to poly($A^+$) RNA from B. oleracea may be due to lower levels of S-protein specific message in whole styles or to more limited homology between the $S_2$-gene and S-genes in Brassica.

Isolation of cDNA clones of S-alleles using the $S_2$-allele specific cDNA clone as a hybridization probe

The homology disclosed herein can be exploited to isolate DNA encoding other S-proteins, without the need for protein microsequencing and oligonucleotide primers, making the isolation of other S-protein coding DNA available to those of ordinary skill in the art.

Total RNA and poly($A^+$) RNA can be isolated by conventional methods using appropriate tissue from a self-incompatible plant of known genotype. Poly($A^+$) RNA from mature styles can then be used to create a cDNA library as exemplified herein in the phage vector λgt10. The protocol of differential screening described above can then be used to obtain mature style specific cDNA clones.

The cDNA NA-2-1 clone insert described above can then be used as a hybridization probe, for example, in a colony hybridization screen. The plaques selected as mature style specific cDNA clones can be picked to agarose plates for regrowth and replicated onto nitrocellulose filters. The NA-2-1 877bp insert can be labelled with $^{32}$P- by nick translation, end labelling or random priming. Plaques which hybridized to the labelled NA-2-1 insert can then be selected for further study. These clones contain cDNA which encodes the desired S-protein although control screening to eliminate false positives is recommended. Standard hybridization conditions for screens of this type have been described (Maniatis et al., 1982). We have recently used this screening procedure to isolate several cDNA clones from cDNA libraries prepared from L. peruvianum ($S_1S_3$) stylar mRNA.

Isolation of chromosomal S-genes using the $S_2$-allele specific cDNA clone as a hybridization probe

DNA can be isolated from a self-incompatible plant of known genotype by conventional methods as for example those described by Rivin, C. J. et al.

0222526

(1982) in <u>Maize</u> <u>for</u> <u>Biological</u> <u>Research</u> (W. F. Sheridan, ed.) pp. 161-164, Plant Mol. Biol. Assn. Charlottesville, Virginia; and Mazure, B. J. and Chui, C.-F. (1985). A genomic DNA library can then be constructed in an appropriate vector. This involves cleaving the genomic DNA with a restriction endonuclease, size selecting DNA fragments and inserting these fragments into a cloning site of the chosen vector. A description of the construction of a <u>Nicotiana</u> <u>tabacum</u> genomic library in the phage λ has been given by Mazure, B. J. and Chui, C.-F. (1985).

The $^{32}$P-labelled cDNA NA-2-1 clone insert described above can be used as a hybridization probe, for example in a filter hybridization screen. An appropriate microorganism is infected with the phage λ containing the genomic library. The infected organisms can be plated on agarose at a concentration of several thousand plaque forming units/plate and replicated onto nitrocellulose filters. The labelled probe can then be applied to the filter and allowed to hybridize. Plaques that show hybridization to the probe are selected, replated and rehybridized until a pure phage is isolated. DNA from selected phage can then be purified, restricted, separated on agarose gels and transferred by blotting to nitrocellulose filters. These filters can then be reprobed with the labelled cDNA <u>S</u>$_2$-allele probe to identify those restriction fragments that contain <u>S</u>-protein coding sequences. Standard hybridization conditions for such screens have been described (Maniatis <u>et</u> <u>al</u>., 1982).

<u>Synthesis</u> <u>of</u> <u>S-protein</u> <u>in</u> <u>heterologous</u> <u>in</u> <u>vivo</u> <u>expression</u> <u>systems</u>

The <u>S</u>-protein DNA coding sequences whose isolation is described herein can be used to direct synthesis of significant amounts of active <u>S</u>-protein.

The DNA encoding the <u>S</u>-protein can be inserted into a recombinant vector so that it is under the control of its own regulatory sequences, an endogenous regulatory region of the vector or an inserted regulatory region by conventional recombinant DNA techniques. The choice of recombinant vector is not crucial. A partial list of vectors includes lambda or M13 bacteriophage, Ti or Ri-plasmids of <u>Agrobacterium</u>, pBR322 derived plasmids, and plant viral vectors such as brome mosaic virus (BMV) or tobacco mosaic virus (TMV). An appropriate host microorganism or plant cell is then transformed with the vector containing <u>S</u>-protein coding sequences. Transformed organisms or cells

-21-

are selected by conventional means and assayed for the expression of active S-protein, for example as in an in vitro pollen tube inhibition assay or by immunoassay. Transformants which produce active protein can then be grown in liquid medium for an appropriate time to allow synthesis of S-protein which is then isolated and subject to further purification, if necessary. S-protein sequences can be maintained on the vector or integrated into the chromosomal DNA of the host, where the S-protein sequences will be flanked by DNA sequences of the host.

Yeast expression systems are particularly useful for the expression of plant proteins since correct post-translational processing of plant proteins has been observed in such systems. Detailed descriptions of the expression of plant proteins in yeast are given in Rothstein, S.J. et al. (1984) Nature 308:662-665; Langridge, P. et al. (1984) EMBO J. 3:2467-2471; Edens, L. et al. (1984) Cell 37:629-633; and Cramer, J.A. et al. (1985) Proc. Natl. Acad. Sci. 82:334-338.

Alternatively, plant proteins can be expressed using similar techniques in bacteria as exemplified in Edens, L. et al. (1982) Gene 18:1-12, which described the expression of the plant protein thaumatin in Escherichia coli. When a bacterial system is employed, the DNA encoding the S-protein should be free of introns, as will be the case with cDNA.

While the presence of a complete signal sequence is not essential to obtain expression of active protein in either yeast or bacteria, more efficient protein synthesis has been observed in yeast when signal sequences are present (Edens, L. et al., 1984).

-22-

Example 1:  Purification of 32K $S_2$-protein from Nicotiana alata styles

Flowers from N. alata (genotype $S_2S_3$) were emasculated at the onset of petal coloration. Two days later, the fully mature styles were removed and stored at -70°C. (Styles refer to the style and stigma which were removed together; ovary is not included.) Frozen styles (3g) were ground to a fine powder in liquid nitrogen using a mortar and pestle; this was followed by further grinding in 50ml of extracting buffer (50mm Tris-HCl, pH 8.5, 1mM $CaCl_2$, 20mM NaCl, 1mM DTT, 10mM EDTA and 1% (w/w) insoluble polyvinylpyrollidone. The homogenate was centrifuged (12,000g; 15 minutes) and the supernatant (11ml) was collected.

Prior to ion exchange chromatography the style extract (11ml) was equilibrated with $NH_4HCO_3$ (5mM, pH 8.6), NaCl (1mM), $CaCl_2$ (1mM), EDTA (1mM) by passage through a Sephadex G-25 (Trademark, Pharmacia Inc., Uppsala, Sweden) column (1.6cm diameter; 22cm long, void volume 11ml). The first 16ml eluted after the void volume was collected and applied to DEAE-Sepharose (Trademark, Pharmacia Inc., Uppsala, Sweden) (bed volume 26ml, 1.6cm diameter x 13cm long) which was equilibrated with the same ammonium bicarbonate buffer. The column was then washed with this buffer (50ml) before the application of a NaCl gradient (0-0.5M). The $S_2$-protein was present in the unbound fractions which were combined and concentrated to a final volume of 16ml by rotary evaporation at room temperature. The $S_2$-protein was further purified by affinity chromatography using ConA-Sepharose (Trademark, Pharmacia Inc. Uppsala, Sweden) followed by gel filtration.

ConA-Sepharose was washed with 5 volumes of methyl-α-D-mannoside (0.1M) in buffer:sodium acetate (10mM, pH 7), 0.1M NaCl, 1mM $MgCl_2$, 1mM $CaCl_2$, 1mM $MnCl_2$. The washed ConA-Sepharose was then transferred to bicarbonate buffer, $NaHCO_3$ (0.25M, p- 8.8) for 1 hour at room temperature; the bicarbonate buffer was changed 4 times during the 1 hour period. Four volumes of $NaHCO_3$ (0.25M, pH 8.8) containing 0.03% (v/v) glutaraldehyde were added and the ConA-Sepharose was then washed with $NaHCO_3$ (0.1M, pH 8.0), containing 0.5M NaCl, resuspended in buffer: sodium acetate (10mM, pH 7), 0.1M NaCl, 1mM $MgCl_2$, 1mM $CaCl_2$, 1mM $MnCl_2$ and packed into a column (0.8cm diameter, 14cm long). The unbound fraction from DEAE-Sepharose was equilibrated in 10mM acetate buffer, by passing through a G25-Sephadex column equilibrated with 10mM acetate

buffer, then applied to the column. Unbound material was collected, the column washed with 10 volumes of acetate buffer, and the bound material eluted with 0.1M or 0.2M methyl-α-D-mannoside in acetate buffer. Fractions containing $S_2$-protein were identified by sodium dodecyl sulfate polyacrylamide gel electrophoresis (SDS-PAGE), collected and concentrated to 1ml by rotary evaporation. The use of a lower pH buffer represents an improvement over the method described in U.S. Patent Application 615,079, and results in improved yields of purified $S_2$-protein. The protein appears to be more stable at lower pH.

The pooled fraction eluted by 0.1M methyl-α-D-mannoside was applied to a column of Biogel P150 (Trademark, Biorad Laboratories, Richmond, California) to separate the methyl-α-D-mannoside from the $S_2$-protein. (Void volume 13ml, 1.6cm diameter, 36.5cm long equilibrated and run in $NH_4HCO_3$ (10mM, pH8.5), 10mM EDTA, 0.1M NaCl 1mM $CaCl_2$. A further passage through Biogel P2 (Trademark, Biorad Laboratories, Richmond, California) in water was used to remove any trace of methyl-α-D-mannoside. The purified $S_2$-protein was essentially homogenous by the criteria of SDS-PAGE (Figure 2a).

SDS-PAGE was performed according to Laemli, U.K. and Favre, M. (1973) J. Mol. Biol. 80:575-583, using 12.5% (w/v) acrylamide. Samples were reduced in 1.43M 2 mercaptoethanol in sample buffer with heating for 2 minutes in a boiling water bath. After electrophoresis, gels were stained with Coomassie Blue.

Example 2:    N-terminal amino acid sequence of the N. alata $S_2$-protein

N-terminal sequencing was performed using an Applied Biosystems (Pfungstadt, West Germany) Model 470A gas phase sequencer. Approximately 200 μg of purified $S_2$-glycoprotein was applied in aqueous solution to a glass fibre disc and evaporated to dryness. The disc was placed in the reaction cell of the sequencer, the protein was eluted and then subjected to 20 cycles of automated Edman degradation by phenylisothiocyanate procedure. The resultant amino acid phenylthiohydantoin derivatives were identified by HPLC techniques on an IBM-CN column (IBM, Danbury, Connecticut) at 32°C using a sodium acetate-acetonitrile gradient, 20mM sodium acetate (pH 5-5.6) varying from 100%-65% (v/v) over 30 minutes. The identity of derivates was confirmed by comparison to known standard reference compounds.

-24-

Example 3:    Comparison of the deglycosylated $S_2$ genotype associated style glycoprotein with the in vitro translation products of style and ovary poly($A^+$) RNA

Frozen mature styles of Nicotiana alata ($S_2S_3$ genotype) were ground to a fine powder in liquid nitrogen using a mortar and pestle.  Protein was extracted from this tissue and the $S_2$-allele associated glycoprotein was isolated by a combination of ion-exchange and affinity chromatography (U.S. Patent Serial No. 615,079).  This material was deglycosylated using a trifluoromethane sulphonic acid (TFMS) procedure modified for use with small quantities of protein (Edge et al. (1981) Annal. Biochem. 118:131-137).

Purified $S_2$-associated glycoprotein (200µg) was lyophilized in a 10ml glass tube with Teflon-lined screw cap and dried over $P_2O_5$ in a dessicator for 18 hours.  Anisole (60µl) and TFMS (120µl) were added and the tube was flushed with $N_2$ for 30 seconds and sealed.  After 90 minutes at 25°C, 10ml of a 1:9 mixture of n-hexane:diethyl ether, precooled on dry ice, was added.  The solution was placed on dry ice for 60 minutes, centrifuged (500g, 5 minutes, 4°C) and the supernatant discarded.  The pellet was air-dried, resuspended in buffer (300µl) and the pH was adjusted to 6.8 by addition of pyridine:$H_2O$ (1:1).  The sample was boiled for 2 minutes before electrophoresis.

Total RNA was isolated from ovary, green bud style or mature style by conventional methods using guanidinium thiocyanate as a protein denaturant. Oligo(dT)-cellulose chromatography was used to isolate mRNA which is polyadenylated, poly($A^+$) RNA.  This poly($A^+$) RNA (2.0 or 0.5µg) was translated using an amino acid depleted rabbit reticulocyte lysate kit (Amersham, Arlington Heights, Illinois) in the presence of 150mM $K^+$, 1.2 mM $Mg^{2+}$ and tritiated amino acids.  Leucine, lysine, phenylalanine, proline and tyrosine were used at specific activities of 5.4, 3.1, 4.8, 3.8 and 4.0 TBq/mmol, respectively.  The reaction volume was 25µl.  After incubation for 90 minutes at 30°C, RNA was removed by treatment with bovine pancreatic ribonuclease (5µl, 2mg/ml) for 20 minutes at 37°.

The glycosylated and deglycosylated samples of pure $S_2$-allele protein were analyzed by SDS-polyacrylamide gel electrophoresis (SDS-PAGE) using 15% acrylamide.  The gels were stained with Coomassie Blue.

Similarly, the translation products of mature style poly(A$^+$) RNA were separated by SDS-PAGE using 10-15% acrylamide gradient gels.  The products were visualized after treatment of the gel with Amplify (Tracemark, Amersham, Arlington Heights, Illinois) and exposure to X-ray film.  In both cases, molecular weight markers were included in adjacent lanes and visualized with Coomassie Blue.

## Example 4:   Preparation of a cDNA library in bacteriophage λgt10

Poly(A$^+$) RNA was isolated from mature styles of N. alata (genotype $S_2S_3$) as described above and transcribed into double stranded cDNA (Maniatis et al., 1982).  Blunt-ended cDNA was prepared by end repair with DNA polymerase.  EcoRI sites contained in the cDNA were blocked by treatment with EcoRI methylase.  Synthetic EcoRI linkers were then ligated to the double stranded cDNA.  The cDNA was then cloned into the EcoRI site of λgt10 as described by (Huynh, et al. 1985).  This phage was used to infect Escherichia coli C600 and plated.

## Example 5:  Differential screening of mature style cDNA library

Poly(A$^+$) RNA was isolated from mature style, green bud style or ovary of N. alata genotype $S_2S_3$.  Single stranded $^{32}$P-labelled cDNA hybridization probes were prepared by random priming from the individual RNA.  Lambda gt10 containing the mature style library was used to infect E. coli C600 and plated at a density of about 1000 plaque forming units/150mm Petrie plate.  Duplicate nitrocellulose lifts were prepared for hybridization (Maniatis et al., 1982).  The plaques were first screened with labelled cDNA probe from mature style and green bud style.  Plaques that hybridized strongly only to the mature style probe were selected, picked, purified and subjected to a second differential screening using the probes to mature style and ovary.  The resultant plaques represent mature style specific clones.

In these plaque hybridizations, the filters were treated prior to hybridization (prehybridized) for 2 hours and during hybridization for 16 hours at 42°C with 5X Denhardt's solution, 5X SSC (3M NaCl, 0.3M Trisodium citrate), 50g/ml sonicated salmon sperm DNA, 50mM sodium phosphate (pH 6.8), 1mM sodium pyrophosphate, 100µM ATP and 50% deionized formamide.  Probes were used at a specific activity of 4 x 10$^7$ cpm/ml. Filters were washed in a 0.1X SSC solution containing 0.1% SDS (sodium dodecyl sulfate) at 42°C.

Example 6:    Isolation of the cDNA clones specific for the S$_2$-allele associated protein

A set of 24 14-mer oligonucleotides was synthesized corresponding to all possible codon ambiguities at amino acids 4-8 in the N-terminal sequence of the S$_2$-protein (Figure 4). Oligonucleotides were synthesized by the solid-phase phosphoramidite methodology (Beaucage and Caruthers, (1981) Tetrahedron Letters 22:1859) using an Applied Biosystems (Pfungstadt, West Germany) ABI Model 380A DNA synthesizer. The 14-mers were end labelled using T4 kinase in the presence of $^{32}$P-ATP (5000 Ci/mmol). These labelled 14-mers (5µg/ml) were used in three batches of 8 14-mers to prime selective cDNA synthesis using mature style poly(A$^+$) RNA. Reverse transcription reaction volume was 40µl. The reaction contained 0.75mM of dCTP, dGTP, dTTP and dATP, 75µg/ml poly(A$^+$) RNA, 50mM Tris-HCl (pH 8.3), 10mM KCl, 8mM, MgCl$_2$, 0.4mM dithiothreitol, 500 U/ml placental RNAase inhibitor and 500U/ml AMV reverse transcriptase. After incubation at 42°C for 90 minutes, the reactions were stopped by addition of EDTA to 50mM, extracted with phenol:chloroform 1:1 (v/v) and the product, labelled cDNA, was precipitated with ethanol. The pellets were resuspended in 20µl of a solution of 100mM NaOH, 7M urea, and 10mM EDTA. Samples were heated at 90°C for 5 minutes before electrophoresis on an 8% (w/v) acrylamide/7M urea gel. The gel was exposed to X-ray film for 5 minutes, to locate specifically primed cDNA products.

As shown in Figure 5, one of the batches of synthetic 14-mers primed synthesis of a 100 bp nucleotide specific for mature style. This 100 bp nucleotide cDNA band was excised from the gel and eluted overnight with shaking at 37°C in 0.5M ammonium acetate and 1mM EDTA. The elutant was concentrated by butanol extraction, phenol:chloroform extracted and ethanol precipitated. The 100 bp nucleotide was then sequenced using the technique of Maxam and Gilbert (1977), Proc. Natl. Acad. Sci. 74:560. The sequence of this nucleotide corresponding to the -12 to +8 amino acid of the S$_2$-protein is shown in Figure 6.

A 30 bp-long synthetic oligonucleotide probe based on the sequence of the 100 bp cDNA and covering the region -8 to +1 of the corresponding amino acid sequence was prepared as described above. The 30-mer probe was end-labelled with $^{32}$P-ATP. This probe was then used to screen the mature style specific clones obtained by differential screening of the λgt10 library. The hybridization of the $^{32}$P-labelled oligomer probe (4 x $10^7$ cpm/ml) was done as described above except that the formamide concentration was decreased to 20% and the temperature was decreased to 37°C. Filters were washed using 2 x SSC at 37°C. Approximately 100,000 plaques from two separately prepared libraries were screened yielding 5 clones that strongly hybridized with the 30 mer probe. One λgt10 clone, designated NA-2-1, was selected for further study. This clone was found to contain a single 877 bp insert which could be excised from the lambda vector by EcoRI digestion. After sequencing of the NA-2-1 clone, it was found that an error had been made in reading the sequencing gel of the 100bp fragment. The sequence shown in Fig. 6 was used to prepare the 30-mer probe. A comparison of Fig. 6 with Fig. 7 in the corresponding region shows a discrepancy between the two sequences at nucleotide 24 (Fig. 6). The sequence of the 30-mer probe that was used in screening does not therefore exactly correspond to the NA-2-1 clone insert.

Example 7: Nucleotide sequence of NA-2-1 cDNA insert

The excised 877 bp DNA insert was sequenced using the chain termination method (F. Sanger et al. (1977) Proc. Natl. Acad. Sci. USA 74:5463-5467; Sanger et al. (1980) J. Mol. Biol. 143:161-178). Figure 7 shows the sequence of this insert containing the full structural coding region for the $S_2$-protein as well as the partial signal sequence. The bases are numbered in the 5' to 3' direction starting with the first base of the structural coding region. Nucleotides on the 5' side of residue 1 are indicated by negative numbers. These negatively numbered bases are in the signal sequence. The protein amino acid sequence as deduced from the nucleotide sequence is also shown. The deduced sequence for amino acids 1-10 and 12-15 is identical to the sequence determined by protein microsequencing (Figure 4). As noted above, an extra adenine nucleotide was found to be inserted in the protein coding sequence. This was confirmed by protein microsequencing of the protein fragment which corresponded to the coding region in question. Several other regions of the nucleotide sequence were confirmed in a similar manner.

-28-

Example 8: Northern blot analysis

A $^{32}$P-labelled probe was prepared from the cDNA clone (NA-2-1) insert encoding the S$_2$-allele associated protein by random priming.  Aliquots of poly(A$^+$) RNA were fractionated on formaldehyde -1.2% (w/v) agarose gels as described by Maniatis, et al. (1982), except that the gel was run in 20mM morpholinopropane sulfonic acid (pH 7.0), 5mM sodium acetate and 0.1mM EDTA (pH 8.0) as a buffer.  The gel was blotted directly onto nitrocellulose filters using 20X  SSC.  Klenow labelled-HindIII EcoRI lambda fragments were used as molecular weights markers.  Prehybridization and hybridization were carried out at 42° as described for plaque hybridization.

Example 9:  Cloning of the nearly full length S$_2$-protein clone from NA-2-1 into M13mp8

The 877 bp NA-2-1 clone insert was excised from λgt10 with EcoRI restriction endonuclease.  The DNA fragments generated were precipitated with ethanol, dried in vaccuo and resuspended in water, to 0.25 µg DNA/µl.  The DNA fragments (2.5 µg) were then subjected to end repair by incubation at 37°C for 1 hour in 25 µl buffer containing:  2mM each of dATP, dCTP, dGTP and dTTP, 10 units DNA polymerase I (Klenow fragment), 50mM Tris-HCl (pH 7.6), 10mM MgCl$_2$ and 10mM dithiothreitol.  The end-repaired fragments were reprecipitated, dried in vaccuo and again suspended in water to 0.25 µg DNA/µl.

The end repaired fragments were inserted into the commercially available vector M13mp8 which had been cut with SmaI restriction endonuclease and dephosphorylated (Amersham, Arlington Heights, Illinois).  Blunt-end ligation was done using 1.25 µg of the end repaired fragments and 20 ng of M13mp8 in a buffer containing 1 U/µl T$_4$ ligase, 1mM ATP 66mM Tris-HCl (pH 7.6), 5mM MgCl$_2$ and 5mM dithiothreitol.  The ligation mixture (total volume of 20 µl) was incubated overnight at 4°C.

The ligation mixture (10 µl) was then used to transform 0.2 ml of competent E. coli JM101 cells (Messing, J. et al. (1981) Nucleic Acids Res. 9:309).  Clones containing the 877 bp S$_2$-protein DNA fragment were using the purified 877 bp S$_2$-clone insert labeled with $^{32}$P by random priming as a hybridization probe. DNA was purified from one of the selected clones and a DNA molecule designated pAEC5 was isolated which consisted of the 877 bp fragment inserted in the SmaI site of M13mp8.

Example 10: Isolation and sequencing of a full-length $S_2$-protein cDNA clone

Mature style poly($A^+$) RNA was used to prepare a second cDNA library in λgt10. The library was constructed according to a method described by Okayama et al. (1982) Mol. Cell Biol. 2:161-170, which was designed to optimize isolation of full-length cDNA clones. A library containing 20,000 plaques was obtained from 5μg of poly($A^+$) RNA. This library was screened as described in Example 6 using the 30-bp long synthetic oligonucleotide probe as well as the 877 bp cDNA insert from the NA-2-1 clone of Example 7. One clone, designated NA-2-2, which hybridized to both probes, was selected for further study.

The NA-2-2 cDNA insert was sequenced using the same methods employed to sequence the NA-2-1 insert. Figure 9 shows the sequence of the NA-2-2 cDNA insert which contains the full structural coding region for the mature $S_2$-protein which is identical to that of the NA-2-1 except that there was no extra adenine nucleotide in the NA-2-2 clone sequence. The NA-2-2 clone also encodes the full signal sequence, which extends 22 amino acids on the N-terminal end of the mature protein. The derived amino acid sequence of the signal peptide of both NA-2-1 and NA-2-2 is identical up to amino acid -18. The reason for the discrepancy in sequence between the two clones is believed to be the result of a sequencing artifact. The two clones are different in the length of their 3' untranslated sequence. They are identical to the polyadenylation site in clone NA-2-2. The NA-2-1 clone contains an extra 50 nucleotides before the poly(A) tail.

Those skilled in the art will appreciate that the invention described herein and the methods of isolation and identification specifically described are susceptible to variations and modifications other than as specifically described. It is to be understood that the invention includes all such variations and modifications which fall within its spirit and scope.

**0222526**

WE CLAIM:

1. A recombinant vector comprising a DNA sequence encoding an $\underline{S}$-protein of a self-incompatible plant.

2. The recombinant vector of claim 1 wherein the self-incompatible plant displays gametophytic self-incompatibility.

3. The recombinant vector of claim 1 wherein the self-incompatible plant displays sporophytic self-incompatibility.

4. The recombinant vector of claim 2 wherein the self-incompatible plant is of the family <u>Solanacae</u>.

5. The recombinant vector of claim 4 wherein the self-incompatible plant is of the genus <u>Lycopersicon</u>.

6. The recombinant vector of claim 4 wherein the self-incompatible plant is of the genus <u>Nicotiana</u>.

7. The recombinant vector of claim 3 wherein the self-incompatible plant is of the family <u>Cruciferae</u>.

8. The recombinant vector of claim 7 wherein the self-incompatible plant is a member of the genus <u>Brassica</u>.

9. The recombinant vector of claim 1 wherein the self-incompatible plant is <u>Nicotiana</u> <u>alata</u>.

10. The recombinant vector of claim 1 wherein the self-incompatible plant is <u>Lycopersicon peruvianum</u>.

11. The recombinant vector of claim 1 wherein the self-incompatible plant is <u>Brassica oleracea</u>.

-31-

12. The recombinant vector of claim 1 wherein the $S$-protein is immunologically cross-reactive with antibody to the $S_2$-protein of Nicotiana alata.

13. The recombinant vector of claim 1 wherein the $S$-protein is substantially homologous to the $S_2$-protein of Nicotiana alata.

14. The recombinant vector of claim 1 wherein the $S$-protein is the $S_2$-protein of Nicotiana alata.

15. The recombinant vector of claim 14 wherein the vector is λgt10.

16. The recombinant vector of claim 14 wherein the vector is M13mp8.

17. The recombinant vector of claim 1 wherein the $S$-protein is the $S_1$-protein of Lycopersicon peruvianum.

18. The recombinant vector of claim 1 wherein the $S$-protein is the $S_3$-protein of Lycopersicon peruvianum.

19. A recombinant DNA molecule comprising a DNA sequence which encodes an $S$-protein of a self-incompatible plant.

20. The recombinant DNA molecule of claim 19 wherein the $S$-protein is the $S_2$-protein of Nicotiana alata.

21. The recombinant DNA molecule of claim 20 which further comprises the promoter sequence of the $S_2$-allele of Nicotiana alata.

22. The recombinant DNA molecule of claim 19 wherein the $S$-protein is the $S_1$-protein of Lycopersicon peruvianum.

23. The recombinant DNA molecule of claim 19 wherein the $S$-protein is the $S_3$-protein of Lycopersicon peruvianum.

24. A recombinant DNA molecule comprising a DNA sequence which encodes a signal sequence of an S-protein of a self-incompatible plant.

25. The recombinant DNA molecule of claim 24 wherein the signal sequence is that of the $S_2$-protein of Nicotiana alata or an S-protein substantially homologous thereto.

26. The recombinant DNA molecule of claim 25 wherein the signal sequence is encoded by DNA comprising the nucleotide sequence 5'-ATG TCT AAA TCA CAG CTA ACG TCA GTT TTC TTC ATT TTG CTT TGT GCT CTT TCA CCG ATT TAT GGG-3'.

27. A recombinant DNA molecule which comprises a promoter sequence of an S-allele of a self-incompatible plant.

28. The recombinant DNA molecule of claim 27 wherein the S-allele is the $S_2$-allele of Nicotiana alata.

29. A method for isolating and identifying an S-gene comprising the step of screening clones which comprise DNA from a self-incompatible plant with a hybridization probe, the DNA sequence of which is based on the DNA sequence encoding the $S_2$-protein of Nicotiana alata.

30. The method of claim 28 wherein the clones comprise DNA from a genomic DNA library of a self-incompatible plant.

31. The method of claim 28 wherein the clones comprise DNA from a cDNA library which is prepared by reverse transcription on a template of mRNA of a self-incompatible plant.

32. A method of making a recombinant vector according to any of claims 1 to 18, said method including the step of inserting into a DNA vector a DNA molecule according to any of claims 19 to 28.

FIG. 1

# FIG. 2

A

32K after
glycoprotein TFMS

B

OVARY   GREEN   MATURE
         BUD
        STYLE   STYLE

1    2          2.0 0.5 2.0 0.5 2.0 0.5 ug

43K

30K

20K

27K

# FIG. 3

## TISSUES OF N.ALATA S₂S₃

P L O A S

P=PETAL
L=LEAF
O=OVARY
A=ANTHER
S=STYLE

94

67

43

30

20

← S₂
PROTEIN

# FIG. 4: N-terminal amino acid sequence of S-proteins

Lycopersicon peruvianum

| | 1 | 2 | 3 | 4 | 5 | 6 | 7 | 8 | 9 | 10 | 11 | 12 | 13 | 14 | 15 |

$S_3$-genotype associated protein  Asp-Phe-Asp-Tyr-Leu-Gln-Leu-Val-Leu-Gln- X -Pro-Arg-Ser-Phe-

$S_1$-genotype associated protein  Tyr-Phe-Glu-Tyr-Leu-Gln-Leu-Val-Leu-Gln- X -Pro-Thr-Thr-Phe-

Nicotiana alata

| | 1 | 2 | 3 | 4 | 5 | 6 | 7 | 8 | 9 | 10 | 11 | 12 | 13 | 14 | 15 |

$S_2$-genotype associated protein  Ala-Phe-Glu-Tyr-Met-Gln-Leu-Val-Leu-Thr-Trp-Pro-Ile-Thr-Phe-

$S_6$-genotype associated protein  Ala-Phe-Glu-Tyr-Met-Gln-Leu-Val-Leu-Gln-Trp-Pro-Thr-Ala-Phe-

# FIG. 6: Partial nucleotide sequence of 100 bp cDNA fragment

```
-12 -11 -10  -9  -8  -7  -6  -5  -4  -3
Phe Ile Leu Leu Cys Ala Leu Ser Pro Ile
TTC ATT TTG CTT TGT GCT CTT TCG CCG ATT

 -2  -1   1   2   3   4   5   6   7   8
Tyr Gly Ala Phe Glu Tyr Met Gln Leu Val
TAT GGG GCT TTC GGG TAC ATG CAG CTC GT
```

30 mer probe sequence

3'-GAA ACA CGA GAA AGC GGC TAA ATA CCC CGA-5'

FIG. 5

167 166 165

GREEN MATURE
BUD
OVARY STYLE STYLE

LANES: 1 2 3 4 5 6

0222526

# FIG. 7: Nucleotide sequence of the near full-length cDNA coding for the 32K molecular weight $S_2$-protein of _Nicotiana alata_.

```
Pro Ala Ser Lys Pro Pro Ala Lys Leu Asp Arg Leu Gln Leu Thr Ser Val Phe Phe Ile
CCG GCA TCA AAA CCT CCC GCC AAG CTG GAC AGA CTC CAG CTA ACG TCA GTT TTC TTC ATT
-90       -80       -70       -60       -50       -40

Leu Leu Cys Ala Leu Ser Pro Ile Tyr Gly Ala Phe Glu Tyr Met Gln Leu Val Leu Thr
TTG CTT TGT GCT CTT TCA CCG ATT TAT GGG GCT TTC GAG TAT ATG CAA CTC GTG TTA ACA
-30       -20       -10        1       10       20       30

Trp Pro Ile Thr Phe Cys Arg Ile Lys His Cys Glu Arg Thr Pro Thr Asn Phe Thr Ile
TGG CCA ATC ACT TTT TGC CGC ATT AAG CAT TGC GAA AGA ACA CCA ACA AAC TTT ACG ATC
          40        50        60        70        80        90

His Gly Leu Trp Pro Asp Asn His Thr Thr Met Leu Asn Tyr Cys Asp Arg Ser Lys Pro
CAT GGG CTT TGG CCG GAT AAC CAC ACC ACA ATG CTA AAT TAC TGC GAT CGC TCC AAA CCC
          100       110       120       130       140       150

Tyr Asn Met Phe Thr Asp Gly Lys Lys Lys Asn Asp Leu Asp Glu Arg Trp Pro Asp Leu
TAT AAT ATG TTC ACG GAT GGA AAA AAA AAA AAT GAT CTG GAT GAA CGC TGG CCT GAC TTG
          160       170       180    ·     190       200       210

Thr Lys Thr Lys Phe Asp Ser Leu Asp Lys Gln Ala Phe Trp Lys Asp Glu Tyr Val Lys
ACC AAA ACC AAA TTT GAT AGT TTG GAC AAG CAA GCT TTC TGG AAA GAC GAA TAC GTA AAG
          220       230       240       250·      260       270

His Gly Thr Cys Cys Ser Asp Lys Phe Asp Arg Glu Gln Tyr Phe Asp Leu Ala Met Thr
CAT GGC ACG TGT TGT TCA GAC AAG TTT GAT CGA GAG CAA TAT TTT GAT TTA GCC ATG ACA
          280       290       300       310       320       330

Leu Arg Asp Lys Phe Asp Leu Leu Ser Ser Leu Arg Asn His Gly Ile Ser Arg Gly Phe
TTA AGA GAC AAG TTT GAT CTT TTG AGC TCT CTA AGA AAT CAC GGA ATT TCT CGT GGA TTT
          340       350       360       370       380       390

Ser Tyr Thr Val Gln Asn Leu Asn Asn Thr Ile Lys Ala Ile Thr Gly Gly Phe Pro Asn
TCT TAT ACC GTT CAA AAT CTC AAT AAC ACG ATC AAG GCC ATT ACT GGA GGG TTT CCT AAT
          400       410       420       430       440       450

Leu Thr Cys Ser Arg Leu Arg Glu Leu Lys Glu Ile Gly Ile Cys Phe Asp Glu Thr Val
CTC ACG TGC TCT AGA CTA AGG GAG CTA AAG GAG ATA GGT ATA TGT TTC GAC GAG ACG GTG
          460       470       480       490       500       510

Lys Asn Val Ile Asp Cys Pro Asn Pro Lys Thr Cys Lys Pro Thr Asn Lys Gly Val Met
AAA AAT GTG ATC GAT TGT CCT AAT CCT AAA ACG TGC AAA CCA ACA AAT AAG GGG GTT ATG
          520       530       540       550       560       570

Phe Pro ***
TTT CCA TGA TTAATAATATTTGTTTTATTGCATTATGCCATGTAAAAAAAAAATTCAAAACCTCAAGTATAAACGTG
          580       590       600       610       620       630       640

TAATCAAGACTATTAAGCACGCACTTATTGAAGACTACACTCGGAAGAATAAGCAAAATTCTTATCAATTTATGGAAATC
          656       666       676       686       696       706       716       726

GTTATTAAAAAAAAAAAAAAAAAAAAAAGGGGGGACGGACTGGGAACGGTTCTTCGGGGTCCCGG
          736       746       756       766       776       786
```

# FIG. 8

0222526

**FIG. 9**: Nucleotide sequence of the full-length cDNA coding for the 32K molecular weight $S_2$-protein of *Nicotiana alata*.

```
                                Met Ser Lys Ser Gln Leu Thr Ser Val Phe Phe Ile
                         GACGGA ATG TCT AAA TCA CAG CTA ACG TCA GTT TTC TTC ATT
                          -70         -60         -50         -40

Leu Leu Cys Ala Leu Ser Pro Ile Tyr Gly Ala Phe Glu Tyr Met Gln Leu Val Leu Thr
TTG CTT TGT GCT CTT TCA CCG ATT TAT GGG GCT TTC GAG TAT ATG CAA CTC GTG TTA ACA
-30         -20         -10          1          10          20          30

Trp Pro Ile Thr Phe Cys Arg Ile Lys His Cys Glu Arg Thr Pro Thr Asn Phe Thr Ile
TGG CCA ATC ACT TTT TGC CGC ATT AAG CAT TGC GAA AGA ACA CCA ACA AAC TTT ACG ATC
            40          50          60          70          80          90

His Gly Leu Trp Pro Asp Asn His Thr Thr Met Leu Asn Tyr Cys Asp Arg Ser Lys Pro
CAT GGG CTT TGG CCG GAT AAC CAC ACC ACA ATG CTA AAT TAC TGC GAT CGC TCC AAA CCC
            100         110         120         130         140         150

Tyr Asn Met Phe Thr Asp Gly Lys Lys Lys Asn Asp Leu Asp Glu Arg Trp Pro Asp Leu
TAT AAT ATG TTC ACG GAT GGA AAA AAA AAA AAT GAT CTG GAT GAA CGC TGG CCT GAC TTG
            160         170         180         190         200         210

Thr Lys Thr Lys Phe Asp Ser Leu Asp Lys Gln Ala Phe Trp Lys Asp Glu Tyr Val Lys
ACC AAA ACC AAA TTT GAT AGT TTG GAC AAG CAA GCT TTC TGG AAA GAC GAA TAC GTA AAG
            220         230         240         250         260         270

His Gly Thr Cys Cys Ser Asp Lys Phe Asp Arg Glu Gln Tyr Phe Asp Leu Ala Met Thr
CAT GGC ACG TGT TGT TCA GAC AAG TTT GAT CGA GAG CAA TAT TTT GAT TTA GCC ATG ACA
            280         290         300         310         320         330

Leu Arg Asp Lys Phe Asp Leu Leu Ser Ser Leu Arg Asn His Gly Ile Ser Arg Gly Phe
TTA AGA GAC AAG TTT GAT CTT TTG AGC TCT CTA AGA AAT CAC GGA ATT TCT CGT GGA TTT
            340         350         360         370         380         390

Ser Tyr Thr Val Gln Asn Leu Asn Asn Thr Ile Lys Ala Ile Thr Gly Gly Phe Pro Asn
TCT TAT ACC GTT CAA AAT CTC AAT AAC ACG ATC AAG GCC ATT ACT GGA GGG TTT CCT AAT
            400         410         420         430         440         450

Leu Thr Cys Ser Arg Leu Arg Glu Leu Lys Glu Ile Gly Ile Cys Phe Asp Glu Thr Val
CTC ACG TGC TCT AGA CTA AGG GAG CTA AAG GAG ATA GGT ATA TGT TTC GAC GAG ACG GTG
            460         470         480         490         500         510

Lys Asn Val Ile Asp Cys Pro Asn Pro Lys Thr Cys Lys Pro Thr Asn Lys Gly Val Met
AAA AAT GTG ATC GAT TGT CCT AAT CCT AAA ACG TGC AAA CCA ACA AAT AAG GGG GTT ATG
            520         530         540         550         560         570

Phe Pro ***
TTT CCA TGA TTAATAATATTTGTTTTATTGCATTATGCCATGTAAAAAAAAAATTCAAAACCTCAAGTATAAACGTG
            580         590         600         610         620         630         640

TAATCAAGACTATTAAGCACGCACTTATTGAAGACTAAAAAAAAAAAAAAAAAAAAAAAA
            656         666         676         686         696
```

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:

ATCC  40201
ATCC  40233

EPO Form 1165 07.81 e